# EUROPEAN PATENT SPECIFICATION

(11) **EP 0 586 595 B1**
(45) Date of publication and mention of the grant of the patent: **09.12.1998**
(21) Application number: 92913737.0
(22) Date of filing: 27.05.1992
(51) Int. Cl.: G01N 33/543, G01N 33/53, G01N 30/90

(54) **ASSAY DEVICE**
TESTVORRICHTUNG
DISPOSITIF D'ANALYSE

(30) Priority: 29.05.1991 US 706639
(43) Date of publication of application: 16.03.1994
(60) Divisional application: 98201475.5
(73) Proprietor: SMITHKLINE DIAGNOSTICS, INC., San Jose California 95134-1622 (US)
(72) Inventor: CHANDLER, Howard, M., West Vancouver, BC, V7V-3X9 (CA)
(74) Representative: Ede, Eric
(86) International application number: PCT/US92/04425
(87) International publication number: WO 92/21977

(56) References cited:
- EP-A- 0 269 362
- EP-A- 0 297 292
- EP-A- 0 309 883
- EP-A- 0 415 679

## Description

### BACKGROUND OF THE INVENTION

This invention is directed to test strips for determination of characteristics of samples, unitized housings, and kits incorporating the test strips, and methods of determining the characteristics of samples using the test strips.

Among the many analytical systems used for detection and/or determination of analytes, particularly analytes of biological interest, are chromatographic assay systems. Among the analytes frequently assayed with such systems are:
(1) hormones, such as human chorionic gonadotropin (hCG), frequently assayed as a marker of human pregnancy;
(2) antigens, particularly antigens specific to bacterial, viral, and protozoan pathogens, such as Streptococcus, hepatitis virus, and Giardia;
(3) antibodies, particularly antibodies induced as a result of infection with pathogens, such as antibody to the bacterium Helicobacter pylori and to human immunodeficiency virus (HIV);
(4) other proteins, such as hemoglobin, frequently assayed in determinations of fecal occult blood, an early indicator of gastrointestinal disorders such as colon cancer;
(5) enzymes, such as aspartate aminotransferase, lactate dehydrogenase, alkaline phosphatase, and glutamate dehydrogenase, frequently assayed as indicators of physiological function and tissue damage;
(6) drugs, both therapeutic drugs, such as antibiotics, tranquilizers and anticonvulsants, and illegal drugs of abuse, such as cocaine, heroin, and marijuana; and
(7) vitamins.

Such chromatographic systems are frequently used by physicians and medical technicians for rapid in-office diagnosis and therapeutic monitoring of a variety of conditions and disorders. They are also increasingly used by patients themselves for at-home monitoring of such conditions and disorders.

Among the most important of such systems are the "thin layer" systems in which a solvent moves across a thin, flat absorbent medium.

Among the most important of tests that can be performed with such thin layer systems are immunoassays, which depend on the specific interaction between an antigen or hapten and a corresponding antibody. The use of immunoassays as a means of testing for the presence and/or amount of clinically important molecules has been known for some time. As early as 1956, J.M. Singer reported the use of an immune-based latex agglutination test for detecting a factor associated with rheumatoid arthritis (Singer et al., Am. J. Med. 22:888-892 (1956)).

Among the chromatographic techniques used in conjunction with immunoassays is a procedure known as immunochromatography. In general, this technique uses a disclosing reagent or particle that has been linked to an antibody to the molecule to be assayed, forming a conjugate. This conjugate is then mixed with a specimen and, if the molecule to be assayed is present in the specimen, the disclosing reagent-linked antibodies bind to the molecule to be assayed, thereby giving an indication that the molecule to be assayed is present. The disclosing reagent or particle can be identifiable by color, magnetic properties, radioactivity, specific reactivity with another molecule, or another physical or chemical property. The specific reactions that are employed vary with the nature of the molecule being assayed and the sample to be tested.

Immunochromatographic assays fall into two principal categories: "sandwich" and "competitive," according to the nature of the antigen-antibody complex to be detected and the sequence of reactions required to produce that complex. In general, the sandwich immunochromatographic procedures call for mixing the sample that may contain the analyte to be assayed with antibodies to the analyte. These antibodies are mobile and typically are linked to a label or a disclosing reagent, such as dyed latex, a colloidal metal sol, or a radioisotope. This mixture is then applied to a chromatographic medium containing a band or zone. This band or zone contains immobilized antibodies to the analyte of interest. The chromatographic medium often is in the form of a strip resembling a dipstick. When the complex of the molecule to be assayed and the labelled antibody reaches the zone of the immobilized antibodies on the chromatographic medium, binding occurs and the bound labelled antibodies are localized at the zone. This indicates the presence of the molecule to be assayed. This technique can be used to obtain quantitative or semi-quantitative results.

Examples of sandwich immunoassays performed on test strips are described by U.S. Patent No. 4,168,146 to Grubb et al. and U.S. Patent No. 4,366,241 to Tom et al. both of which are incorporated herein by this reference.

A device for chemical analyses especially in the fields of medicine and agriculture is proposed in US-A-4 717 656. The device includes a foldable continuous sheet divided into segments carrying sample or reagent sites, the former being contactable by the latter by appropriate folding action to permit overlapping. Separation of excess reagent is provided for by e.g. an additional absorbing medium. The device described there differs essentially from prior art chromatographic devices through the fact that it is not based upon any diffusion principle.

An analytical device for use in immunoassays is described in GB-A-2 204 398 in which a hollow casing houses a dry porous carrier containing a mobile specific binding reagent for an analyte possibly present in a sample to be applied to the carrier. The carrier also carries a detection zone containing immobilised specific binding reagent for the same analyte. As the sample moves through the carrier towards the detection zone labelled antibody can be picked up by analyte to form a sandwich in that zone with the immobilized antibody. Various arrangements are proposed but all include a single, linearly contiguous, element for fluid flow to provide a single-element immunochromatographic test device for detection of an analyte by either a sandwich or a competitive immunoassay.

In addition to immunochromatographic assays, it is also known to use enzyme-based chromatographic assays. These techniques are roughly analogous to immunochromatographic assays, but use an enzymatically catalyzed reaction instead of an antigen-antibody reaction. The enzymatically catalyzed reaction frequently generates a detectable product. Other analogous chromatographic assays are known.

Although useful, currently available chromatographic techniques using test strips have a number of drawbacks. Many samples, such as fecal samples, contain particulate matter that can clog the pores of the chromatographic medium, greatly hindering the immunochromatographic process. Other samples, such as blood, contain cells and colored components that make it difficult to read the test. Even if the sample does not create interference, it is frequently difficult with existing chromatographic test devices to apply the sample to the chromatographic medium so that the sample front moves uniformly through the chromatographic medium to insure that the sample reaches the area where binding is to occur in a uniform, straight-line manner.

Sample preparation and waste generation are responsible for other problems with currently available devices and techniques for immunochromatography. The increased prevalence of diseases spread by infected blood and blood fractions, such as AIDS and hepatitis, has exacerbated these problems. It is rarely possible to apply a sample (such as feces) or a sampling device (such as a throat swab) directly to the chromatographic medium. Several extraction and pretreatment reactions are usually required before the sample can be applied to the chromatographic medium. These reactions are typically carried out by the physician or technician performing the test in several small vessels, such as test tubes, or microfuge tubes, requiring the use of transfer devices, such as pipettes. Each of these devices is then contaminated and must be disposed of using special precautions so that workers or people who may inadvertently come into contact with the waste do not become contaminated.

Still another limitation on chromatographic devices currently available for use by the clinician or technician is their inability to perform two-directional or two-dimensional chromatography. These techniques have long been known to be powerful analytical tools, but their complexity relative to simple unidirectional chromatography has made it difficult to apply them to test strip devices in the physician's office or a clinical laboratory.

Accordingly, there is a need for an improved chromatographic device for the performance of immunochromatographic assays or other analogous assays. Such a device should be capable of receiving a possibly contaminated sample or a sample preparation device directly so as to eliminate the need for extraction vessels and transfer devices. Such a device, preferably in the form of a test strip, should also be capable of performing immunochromatographic assays on colored samples or samples containing particulates without interference and should be able to deliver the sample to the chromatographic medium uniformly and evenly to improve accuracy and precision of the tests. Additionally, such an improved test strip should be capable of performing two-directional or two-dimensional chromatography when used in clinical laboratories or physicians' offices.

### SUMMARY

We have developed an assay device that meets these needs and provides improved assays for analytes of biological interest, while simplifying the performance of the assay and avoiding contamination.

A chromatographic assay device according to the present invention comprises:
(1) a first opposable component including a sample preparation means adapted to receive a sample to be assayed; and
(2) a second opposable component including a chromatographic medium, wherein the first and second opposable components can be brought into opposition so as to cause sample liquid from the sample preparation means to be applied to the component including the chromatographic medium so as to contact said chromatographic medium.

The sample preparation means can contain at least one reagent for treatment of the sample before the sample is applied to the chromatographic medium. Preferably, the reagent is an extraction reagent to extract analyte from the sample.

The first and second opposable components can each further include engaging means that secure the first and second opposable components in opposition. The first and second opposable components can be joined by a hinge.

Typically, the chromatographic medium has first and second ends and the device further comprises conducting means in operable contact with the first end of the chromatographic medium.

Preferably, the chromatographic medium further includes a detection zone substantially smaller than the chromatographic medium. More preferably, the detection zone contains a first specific binding partner to the analyte immobilized thereto. If the analyte is an antigen or hapten, the first specific binding partner can be an antibody to the antigen or hapten. If the analyte is an antibody, the first specific binding partner can be a hapten or antigen capable of being bound specifically by the antibody.

Preferably, the chromatographic medium further includes a control zone substantially smaller than the chromatographic medium and separate from the detection zone. Typically, the control zone contains analyte immobilized thereto, but other arrangements are possible, depending on the chemical nature of the analyte.

Preferably, the device further comprises an absorbing means in operable contact with the second end of the chromatographic medium to enhance the flow of the sample through the chromatographic medium.

The sample preparation means can further contain a specific binding partner for the analyte labelled with a detectable label in a form that can be resolubilized by the addition of an aqueous liquid to the sample preparation means.

In the device, at least one of the first and second opposable components can include an aperture therein for viewing of at least a portion of the chromatographic medium.

A test kit can comprise the chromatographic assay device described above and a specific binding partner for the analyte labelled with a detectable label. Preferably, the label is a visually detectable label.

A method for detecting and/or determining an analyte in a sample using this assay device can comprise the steps of:
(1) applying the sample to the sample preparation means of the chromatographic assay device;
(2) applying a detection reagent to the sample preparation means, the detection reagent comprising at least one component capable of binding specifically to analyte present in the sample;
(3) bringing the first and second opposable components into opposition so that the sample preparation means applies the sample and the detection reagent to the chromatographic medium;
(4) allowing the sample and the detection reagent to move through at least a portion of the chromatographic medium so that the detection reagent gives a detectable indication of the presence and/or quantity of the analyte; and
(5) observing and/or measuring the detection reagent in at least a portion of the chromatographic medium in order to detect and/or determine the analyte.

If the sample preparation means further contains a specific binding partner for the analyte labelled with a detectable label in a form that can be resolubilized by the addition of an aqueous liquid to the sample preparation means, an assay method can comprise the steps of:
(1) applying the sample as an aqueous liquid to the sample preparation means of the chromatographic assay device, thereby resolubilizing the specific binding partner for the analyte with the detectable label so that the labelled specific binding partner can bind specifically to analyte present in the sample;
(2) bringing the first and second opposable components into opposition so that the sample preparation means applies the sample and the labelled specific binding partner to the chromatographic medium;
(3) allowing the sample and the labelled specific binding partner to move through at least a portion of the chromatographic medium so that the labelled specific binding partner gives a detectable indication of the presence and/or quantity of the analyte; and
(4) observing and/or measuring the labelled specific binding partner in at least a portion of the chromatographic medium in order to detect and/or determine the analyte.

Yet another embodiment of an assay device according to the present invention comprises:
(1) a first opposable component including:
   (a) a chromatographic medium having first and second ends;
   (b) a first conducting means in operable contact with the first end of the chromatographic medium; and
   (c) a second conducting means in operable contact with the second end of the chromatographic medium;
(2) a second opposable component hingedly attached to the first opposable component including:
   (a) a first absorbing means; and
   (b) a first application means separated from the first absorbing means; and
(1) a third opposable component hingedly attached to the first opposable component including:
   (a) a second absorbing means; and
   (b) a second application means separated from the second absorbing means.

In this device, bringing the first and second opposable components into opposition causes the first absorbing means to come into operable contact with the second conducting means to withdraw fluid from the chromatographic medium, and causes the first application means to come into operable contact with the first conducting means to apply fluid to the chromatographic medium, so that a first liquid applied to the first application means is drawn through at least a portion of the chromatographic medium. Also, subsequently, bringing the first and third opposable components into opposition causes the second absorbing means to come into operable contact with the first conducting means to withdraw fluid from the chromatographic medium and causes the second application means to come into operable contact with the second conducting means to apply fluid to the chromatographic medium so that a second liquid applied to the second application means is drawn through at least a portion of the chromatographic medium overlapping the portion of the chromatographic medium through which the first liquid is drawn. The first, second, and third opposable components are in such a configuration that, that when the third opposable component is brought into opposition with the first opposable component, the second opposable component can be folded over the first and third opposable components to form a cover.

Typically, in this embodiment, the first application means can contain a first specific binding partner for the analyte in a form that can be resolubilized by the application of an aqueous sample to the application means. This embodiment is particularly useful for indirect labeling, in which the first specific binding partner to the analyte is not itself labeled. Rather, a secondary specific binding partner specific for the first binding partner is labeled. This secondary specific binding partner is applied to the second application means to indirectly label the analyte.

Another embodiment of an assay device according to the present invention comprises:
(1) a first opposable component including:
   (a) a chromatographic medium having first and second ends;
   (b) a conducting means in operable contact with the first end of the chromatographic medium; and
   (3) an absorbing means in operable contact with the second end of the chromatographic medium; and
(2) a second opposable component including:
   (a) a first application means; and
   (b) a second application means;
the first and second application means being positioned on the second opposable component such that they are not in operable contact when the first and second opposable components are not in opposition.

In this device, bringing the first and second opposable components into opposition places the conducting means in operable contact with the first application means and also places the conducting means in operable contact with the second application means, thereby placing the first and second application means in operable contact with each other.

Yet another version of a chromatographic assay device according to the present invention incorporating two application means on the second opposable component comprises:
(1) a first opposable component including:
   (a) a chromatographic medium having first and second ends;
   (b) a conducting means positioned such that it is not in operable contact with the first end of the chromatographic medium with the first opposable component and second opposable component are not in opposition; and
   (c) an absorbing means in operable contact with the second end of the chromatographic medium; and
(2) a second opposable component comprising:
   (a) a first application means; and
   (b) a second application means.

The first and second application means are positioned on the second opposable component such that they are not in operable contact when the first and second opposable components are not in opposition. In this device, bringing the first and second opposable components into opposition places the conducting means in operable contact with the first application means, places the conducting means in operable contact with the second application means, and places the second application means in operable contact with the first end of the chromatographic medium, thereby placing the first and second application means in operable contact with each other to apply the contents of the first and second application means to the chromatographic medium.

Another embodiment of an assay device according to the present invention comprises:
(1) a first opposable component including:
   (a) a chromatographic medium having first and second ends;
   (b) a conducting means in operable contact with the first end of the chromatographic medium;
   (c) an absorbing means in operable contact with the second end of the chromatographic medium; and
   (d) a detector application pad in direct contact with the conducting means and positioned such that it is in indirect contact with the first end of the chromatographic medium; and
   (b) a second opposable component including a sample application pad.

In this device, bringing the first and second opposable components into opposition causes the sample application pad to apply the sample to be tested to the detector application pad and thus to the first end of the chromatographic medium though the conducting means.

A variation of this device can provide superior dynamic range for analytes such as hemoglobin in feces. This variation comprises:
(1) a first opposable component including:
   (a) a chromatographic medium having first and second ends;
   (b) an absorbing means in operable contact with the second end of the chromatographic medium; and
   (c) a detector application pad in direct contact with the first end of the chromatographic medium; and
(2) a second opposable component including a sample application pad.

In this variation, when the first and second opposable components are brought into opposition, the detector application pad and the sample application pad are in contact except for the region of the detector application pad directly adjacent to the first end of the chromatographic medium. Also, bringing the first and second opposable components into opposition causes the sample application pad to apply the sample to be tested to the detector application pad and thus to the first end of the chromatographic medium.

### BRIEF DESCRIPTION OF THE DRAWINGS

These and other features, aspects, and advantages of the present invention will become better understood with reference to the following description, appended claims, and accompanying drawings where:
Figure 1A is a drawing of one version of a two-component chromatographic assay device according to the present invention;
Figure 1B is a drawing of the two-component chromatographic assay device of Figure 1A shown with the two components having been brought into opposition;
Figure 4 is a drawing of yet another version of a two-component assay device according to the present invention incorporating two application means on one of the components;
Figure 5 is a drawing of yet another version of a two-component assay device according to the present invention incorporating a discontinuity between a conducting means and the chromatographic medium that is bridged when the device is closed;
Figure 6A is a drawing of yet another version of a two-component assay device according to the present invention incorporating a detector application pad in operative contact with the chromatographic medium;
Figure 6B is a side view of the two-component assay device of Figure 6A, showing details of the components in opposition;
Figure 7A is a drawing of yet another version of a two-component assay device according to the present invention, generally similar to the version of Figure 6, but with the detector application pad in direct contact with the chromatographic medium;
Figure 7B is a side view of the two-component assay device of Figure 7A, showing details of the components in opposition;
Figure 11A is a drawing of a three-component assay device according to the present invention;
Figure 11B is a side view of the three-component assay device of Figure 11A, showing details of the components in opposition;
Figure 16 is a depiction of an assay device according to the present invention suitable for receiving a swab or similar sampling device and designed for detection of Streptococcus A antigen.

### DESCRIPTION

### Definitions

In the context of this disclosure, the following terms are defined as follows unless otherwise indicated:

Specific Binding Partner: A member of a pair of molecules that interact by means of specific non-covalent interactions that depend on the three-dimensional structures of the molecules involved. Typical pairs of specific binding partners include antigen-antibody, hapten-antibody, hormone-receptor, nucleic acid strand-complementary nucleic acid strand, substrate-enzyme, inhibitor-enzyme, carbohydrate-lectin, biotin-avidin, and virus-cellular receptor.

Operable Contact: Two solid components are in operable contact when they are in contact, either directly or indirectly, in such a manner that an aqueous liquid can flow from one of the two components to the other substantially uninterruptedly, by capillarity or otherwise. "Direct contact" means that the two elements are in physical contact, such as edge-to-edge or front-to-back. "Indirect contact" means that the two elements are not in physical contact, but are bridged by one or more conducting means.

Finite Capacity: An absorbing means has finite capacity when it becomes saturated by liquid received during the normal performance of an assay in the device in which the absorbing means is located. At that point, the absorbing means can release additional liquid absorbed and become at least partially conductive.

Analyte: The term "analyte" includes both the actual molecule to be assayed and analogues and derivatives thereof when such analogues and derivatives bind another molecule used in the assay in a manner substantially equivalent to that of the analyte itself.

Antibody: The term "antibody" includes both intact antibody molecules of the appropriate specificity and antibody fragments (including Fab, F(ab'), and F(ab')₂ fragments) as well as chemically modified intact antibody molecules and antibody fragments, including hybrid antibodies assembled by in vitro reassociation of subunits.

Secondary Specific Binding Partner: An additional specific binding partner that binds to a member of a pair of specific binding partners when the pair of specific binding partners is interacting is designated a secondary specific binding partner. For example, a pair of specific binding partners can comprise Giardia antigen and rabbit anti-Giardia antibody. In that case, the secondary specific binding partner can be goat anti-rabbit IgG antibody. The secondary specific binding partner can be specific for the species, class, or subclass of an antibody specific binding partner to which it binds. Alternatively, when one of the specific binding partners is labelled with biotin, the secondary specific binding partner can comprise a molecule conjugated to avidin.

### I. CHROMATOGRAPHIC ASSAY DEVICES

One aspect of the present invention comprises chromatographic assay devices particularly useful for the assay of analytes in biological samples. These devices are suitable for the direct application of biological samples, without preliminary extraction steps, and are constructed so as to minimize interference with assay results caused by particulates or colored samples.

### A. Two-Component Devices

One embodiment of the assay device of the present invention is a two-component chromatographic assay device operating in one dimension with one-directional flow.

### 1. General Arrangement of Two-Component Device

In general, a two-component chromatographic assay device according to the present invention comprises:
(1) A first opposable component including a sample preparation means adapted to receive a sample to be assayed; and
(2) A second opposable component including a chromatographic medium.

In this device, the first and second opposable components can be brought into opposition when the device is closed so as to cause a sample preparation means to apply the sample to be assayed to the chromatographic medium. In use, the first and second opposable components are typically brought into opposition after a detection reagent is applied to the sample preparation means. When the first and second opposable components are brought into opposition, the sample preparation means applies the sample and detection reagent to the chromatographic medium. After the sample and detection reagent is allowed to traverse at least a portion of the chromatographic medium, so that the detection reagent gives a detectable indication of the presence and/or quantity of the analyte; the detection reagent is then observed and/or measured in at least a portion of the chromatographic medium. This results in detection and/or determination of the analyte.

The detection reagent comprises the first specific binding partner for the analyte as described above; it may comprise additional components.

This process can give a qualitative and/or quantitative indication of the analyte, depending upon the density of the second specific binding partner in the detection zone and the size of the detection zone.

Typically, to achieve results, the assay requires from 30 seconds to 10 minutes, more typically, from 1 to 5 minutes, including any period of incubation of the sample on the sample preparation means, as well as the time required for chromatography itself. Typically, the assay is performed at room temperature, although it can be performed at 4°C or up to 37°C or higher in some cases, depending upon the nature of the analyte and specific binding partners. In some cases, performing the assay at a lower temperature may be desirable to limit degradation, while in other cases, performing the assay at a higher temperature with suitable analytes and specific binding partners may speed up the assay.

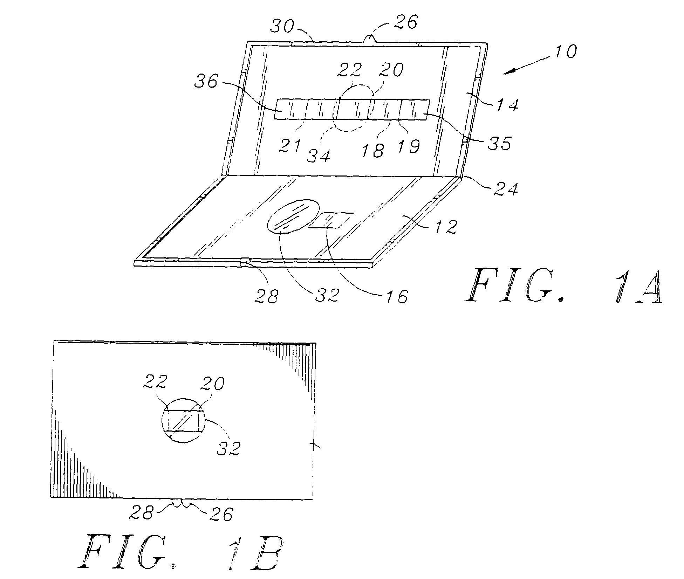

This general arrangement of the chromatographic assay device is shown in Figure 1A. The chromatographic assay device 10 has a first opposable component 12 and a second opposable component 14. The first opposable component 12 includes a sample preparation means 16. The second opposable component 14 contains a chromatographic medium 18. The chromatographic medium has a first end 19 and a second end 21; the chromatographic medium 18 contains a detection zone 20 and a control zone 22. The first opposable component 12 and the second opposable component 14 are joined by a hinge 24 and have locking means 26 and 28 that are engaged when the first opposable component 12 and the second opposable component 14 are brought into opposition. The sealing ridge or gasket 30 is positioned around the perimeter of the first and second opposable components 12 and 14. The second opposable component 14 has a first window 34; optionally, the first opposable component 12 can have a second window 32 to permit viewing of the chromatographic medium 18 from either side.

Figure 1B shows the device 10 after the opposable components 12 and 14 have been brought into opposition. The chromatographic medium 18, including the detection zone 20 and the control zone 22, is visible through window 34.

The device 10 can, optionally, further comprise conducting means 35 in operable contact with the first end 19 of the chromatographic medium 18, as shown in Figure 1A. The conducting means 35 can be a material such as cellulose or other material that can conduct an aqueous liquid without substantially absorbing it. The conducting means 35 can be treated with a surfactant so that the conjugate can be applied more evenly to the chromatographic medium.

The device 10 can further comprise an absorbing means 36 in operable contact with the second end 21 of the chromatographic medium 18 to aid in drawing fluid through the chromatographic medium 18 from the first end 19 toward the second end 21, as shown in Figure 1A.

The sample preparation means can be made of any suitable material, such as, but not limited to, cellulose, paper, nylon, rayon, glass fiber, fleeces, or non-woven synthetic fabrics. The porosity of the sample preparation means can be chosen to filter out cellular or particulate matter in samples such as whole blood or fecal samples. The sample preparation means can contain at least one reagent for treatment of the sample before the sample is applied to the chromatographic medium.

The reagents that can be present in the sample preparation means vary with the sample to be applied to the sample preparation means and with the analyte to be assayed. They can include, but are not limited to, acids or alkalis to adjust the pH, buffers to stabilize the pH, chelating agents such as EDTA or EGTA to chelate metals, hydrolytic enzymes to lyse the cell membrane of animal cells or the cell wall of bacteria to liberate analytes, substrates or co-enzymes for enzymes, and the like. One particularly useful extraction reagent is a mixture of sodium nitrite and acetic acid to generate nitrous acid. The sodium nitrite can be present in dried form on the sample preparation means, and the acetic acid can be added to the sample preparation means after the addition of the sample.

The sample, or optionally, a sampling device such as a throat swab or a microporous filter, can be placed by the operator on the sample preparation means; if needed, other reagents can be added.

The bodies of the first and second opposable components are preferably made of plastic that is impervious to moisture. A suitable plastic is a polycarbonate plastic such as Lexan™. However, other materials, such as paperboard or solid bleached sulfite (SBS), can be used.

Typically, the chromatographic medium, absorbing means, conducting means, application means, and other liquid-receiving components are secured to the bodies of the first and second opposable components by adhesive. Suitable adhesives are well known in the art.

Typically, when two components are in direct contact, they are overlapped with an overlap of about 0.5 to about 3 mm. However, the components can be placed with abutting edges.

The first and second opposable components preferably further comprise engaging means that secure the first and second opposable components in opposition. The engaging means can further comprise locking means.

At least one of the first and second opposable components preferably contains a window or aperture so that the relevant portion of the chromatographic medium can be viewed. Preferably, the window or aperture is located in the second opposable component. Alternatively, both the first and second opposable components can contain a window or aperture to allow viewing of the chromatographic medium from both sides.

A sealing wedge or gasket can be provided around a perimeter of the opposable components to guard against leakage of samples or reagents.

The analyte is detected either by means of a labelled specific binding partner to the analyte or by the use of a labelled secondary specific binding partner for a specific binding partner to the analyte. In most cases, the use of a labelled specific binding partner to the analyte is preferred. The label of the labelled specific binding partner is preferably a visually detectable label, such as a colloidal metal label. Preferably, the colloidal metal label is gold, silver, bronze, iron, or tin; most preferably, it is gold. The preparation of gold-labelled antibodies is described in J. DeMey, "The Preparation and Use of Gold Probes," in Immunocytochemistry: Modern Methods and Applications (J.M. Polak & S. VanNoorden, eds. Wright, Bristol, England, 1986), Ch. 8, pp. 115-145, incorporated herein by this reference. Antibodies labelled with colloidal gold are commercially available, such as from Sigma Chemical Company, St. Louis, Missouri.

Alternatively, other colloidal labels, such as a colloidal sulfur label, can also be used.

Although Applicant does not necessarily intend to be bound by this theory, when an aqueous liquid containing a sample is applied to a resolubilizible specific binding partner labeled with a colloidal metal label, such as colloidal gold, the kinetics of the reaction between the analyte and the labeled specific binding partner are extremely rapid. These rapid kinetics result in the substantially complete labeling of analyte before the combination of the analyte and the labeled specific binding partner is applied to the chromatographic medium. Thus, in a one-directional chromatographic procedure performed with an assay device according to the present invention, what is chromatographed is predominantly the binary complex of the analyte and the corresponding labeled specific binding partner. This allows separation of this complex from contaminants not binding the specific binding partner and improves accuracy of the assay.

In this embodiment, the labelled specific binding partner preferably is present on the sample preparation means in a form that can be resolubilized by the addition of an aqueous liquid to the sample preparation means. Typically, the aqueous liquid is the sample itself. In some cases, particularly where small sample volumes are used, it may be desirable to add additional buffer or other aqueous liquid to the sample preparation means.

In other embodiments discussed below, the labelled specific binding partner can be present on an element of the chromatographic assay device that is separate from the sample preparation means but comes into contact with it during the performance of the assay. In these embodiments, the labelled specific binding partner is preferably present in a resolubilizable form on this element, and is resolubilized when the sample comes into contact with the element. In some cases, the labelled specific binding partner can be resolubilized by the addition of a separate aqueous liquid, distinct from the sample, to the element.

In a less preferred alternative, the visually detectable label can be a colored latex label. It is also possible to use other labels, such as radioactive labels.

The chromatographic medium on the second opposable component is a flat strip. It is typically rectangular, having first and second ends. Throughout this Description, the term "first end" refers to the end of the chromatographic medium at which the sample is applied, and the term "second end" refers to the opposite end. The original direction of flow of the sample is from the first end toward the second end of the chromatographic medium. The chromatographic medium is composed of a material suitable as a medium for thin-layer chromatography of analytes and analyte-antibody conjugates, such as nitrocellulose, nylon, rayon, cellulose, paper, or silica. The chromatographic medium can be pretreated or modified as needed. Typically, the chromatographic medium is translucent, so that colored zones appearing on it as a result of the assay can be viewed from either side.

In some applications, it is preferable to place a second flexible transparent support on the top of the chromatographic medium to regulate the flow of the sample through the membrane and prevent migration over the top of the membrane. Suitable flexible transparent supports include polyethylene, vinyl, Mylar®, and cellophane.

When the chromatographic assay device is to be used for an assay such as a sandwich immunoassay, the chromatographic medium can further comprise a detection zone substantially smaller than the chromatographic medium. This detection zone can contain a second specific binding partner to the analyte immobilized thereto against diffusion. The second specific binding partner can be bound to the analyte by either covalent or non-covalent means. If the analyte to be assayed is an antigen or hapten, the second specific binding partner can be an antibody to the antigen or the hapten. Alternatively, the analyte can be an antibody and the second specific binding partner can be a hapten or an antigen capable of being bound specifically by the antibody.

The chromatographic medium can further comprise a control zone substantially smaller than the chromatographic medium, and separate from the detection zone. The control zone can comprise analyte immobilized thereto non-diffusibly in order to bind labelled antibody that is not bound at the detection zone by the formation of a ternary "sandwich" complex. Any such antibody is bound by the immobilized analyte and forms a detectable zone or band. This provides a check on the operation of the assay and the correct binding of the reagents, as described below. The methods used to bind the second specific binding partner in the detection zone and the analyte in the control zone are well known in the art and need not be described further.

Alternatively, for some analytes, such as carbohydrates, it may be difficult or impossible to fix the analyte stably to the chromatographic medium. In such cases, the control zone can comprise an immobilized zone of antibody specific for the labelled anti-analyte antibody. For example, if the analyte is the Streptococcus A-specific carbohydrate, and the labelled antibody is rabbit IgG specific for Streptococcus A antigen, the control zone can comprise goat antibody to rabbit IgG. In such cases, to prevent complete capture of the labelled anti-analyte antibody in the detection zone at high analyte concentrations and consequent disappearance of the labelled anti-analyte antibody from the control zone, it can be desirable to add labelled antibody not specific for the analyte to the labelled anti-analyte antibody. Such antibody can constitute immunologically indifferent immunoglobulin or an antibody to an analyte not found in the test sample.

Several variations of this device are possible. In one variation, as discussed above, the sample preparation means can further contain a specific binding partner for the analyte labelled with a detectable label in a form that can be resolubilized by the addition of an aqueous liquid to the sample preparation means. The aqueous liquid can be the sample itself. The labelled specific binding partner can be freeze-dried or reversibly precipitated so that it is resolubilized and mobilized by the addition of the sample to the sample preparation means. In this variation, it is not necessary to add the detection reagent to the sample preparation means, as the detection reagent is automatically generated by the addition of the sample to the sample preparation means.

In another variation, the second opposable component can further comprise an absorbing means of finite capacity in operable contact with the first end of the chromatographic medium. The absorbing means is located so that it comes into contact with the sample preparation means when the first and second opposable components are placed into opposition. Thus, the sample is applied to the absorbing means when the first and second opposable components are brought into opposition. This may be useful in controlling the flow of sample into the chromatographic medium so that the chromatographic medium is not overloaded.

In this version, the absorbing means can contain a labelled specific binding partner for the analyte in a form that can be resolubilized, as described above. In this arrangement, the labelled specific binding partner is resolubilized when the first and second opposable components are brought into opposition, applying the sample to the absorbing means. The combination of the sample and the resolubilized labelled specific binding partner than enters the chromatographic medium at its first end.

### b. Device Comprising Two Separate Application Means on Same Element

Yet another embodiment of a chromatographic assay device according to the present invention comprises two separate application means on the same element. These two application means are not in operable contact until they are bridged by a conducting means on the opposing element when the elements are brought into opposition.

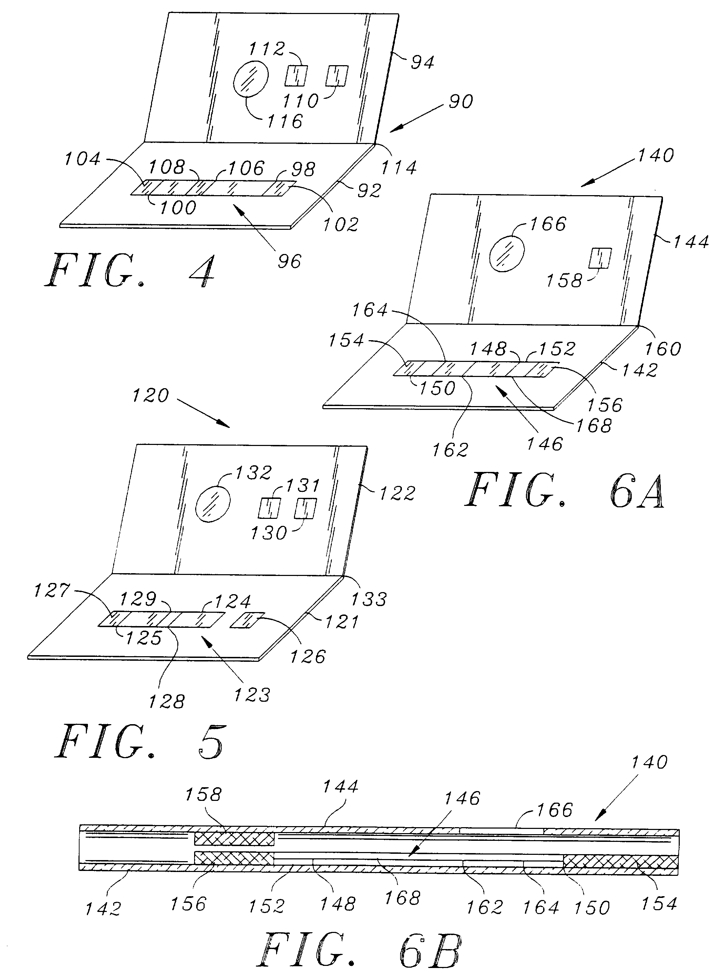

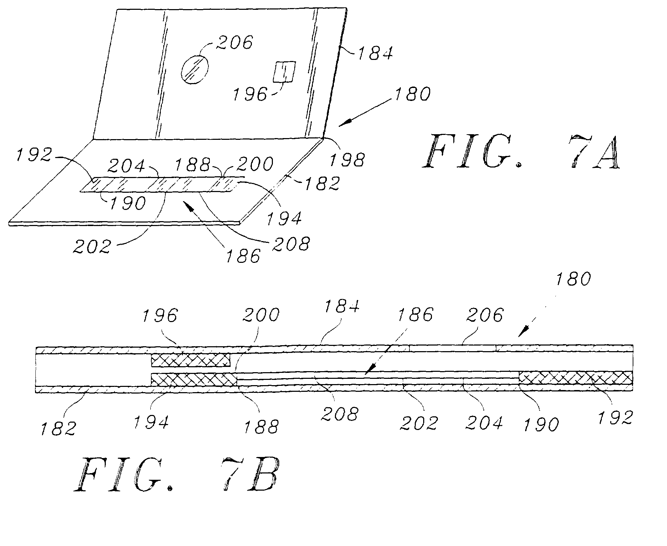

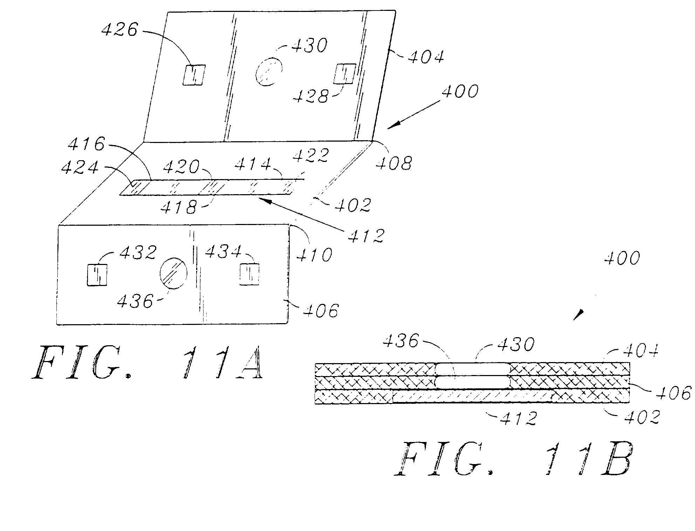

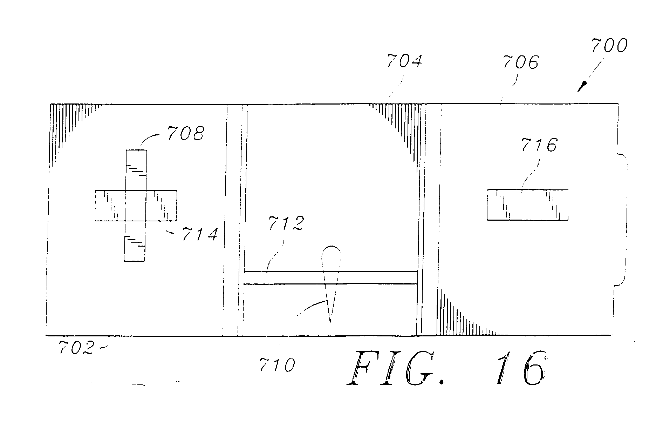

This embodiment of the chromatographic assay device is shown in Figure 4. The chromatographic assay device 90 has a first opposable component 92 and a second opposable component 94. The first opposable component 92 comprises a chromatographic medium 96 having a first end 98 and a second end 100, a conducting means 102 in operable contact with the first end 98, and an absorbing means 104 in operable contact with the second end 100 of the chromatographic medium 96. The chromatographic medium 96 contains a detection zone 106 and a control zone 108. The second opposable component 94 contains a first application means (sample application pad) 110 and a second application means (detector application pad) 112. The first application means 110 and the second application means 112 are not in operable contact until the first opposable component 92 and the second opposable component 94 are brought into opposition. When the first opposable component 92 and the second opposable component 94 are brought into opposition, the first application means 110 and the second application means 112 are bridged by the conducting means 102 so that the contents of the first application means 110 and the second application means 112 are applied to the chromatographic medium 96. The first opposable component 92 and the second opposable component 94 are joined by a hinge 114. The second opposable component 94 contains a window 116 to allow viewing of the chromatographic medium 96.

The first and second application means are positioned on the second opposable component such that they are not in operable contact when the first and second opposable components are not in opposition. Bringing the first and second opposable components into opposition places the conducting means in operable contact with the first application means and also with the second application means, thereby placing the first and second application means in operable contact with each other. Thus, bringing the first and second opposable components into opposition allows the contents of the first and second application means to react and applies the contents of both the first and second application means to the chromatographic medium. Chromatography and detection of the analyte then occur as described above.

The first application means can comprise a sample application pad and the second application means can comprise a detector application pad, to which detecting reagent can be applied. When the first and second opposable components are brought into opposition, the contents of the sample application pad and the detector application pad are applied to the chromatographic medium via the conducting means.

Preferably, the second application means (detector application pad) contains a specific binding partner for the analyte labelled with a detectable label in a form that can be resolubilized by the addition of an aqueous liquid to the second application means. The aqueous liquid is typically the sample itself, which resolubilizes the labelled specific binding partner when the first and second opposable components are brought into opposition. In some assays, it may be desirable to add a separate reconstituting aqueous liquid to the detector application pad. Alternatively, the labelled specific binding partner can be applied in liquid form to the second application means.

A further variation of this device incorporates a gap or discontinuity between the conducting means and the chromatographic medium so that the path of fluid flow is from the first application means through the conducting means, then through the second application means, and finally through the chromatographic medium.

This variation of the device is shown in Figure 5. The chromatographic assay device 120 has a first opposable component 121 and a second opposable component 122. The first opposable component 121 comprises a chromatographic medium 123 having a first end 124 and a second end 125, a conducting means 126 not in operable contact with the first end 124 of the chromatographic medium 123 when the device 120 is in open position, and an absorbing means 127 in operable contact with the second end 125 of the chromatographic medium 123. The chromatographic medium 123 contains a detection zone 128 and a control zone 129. The second opposable component 122 contains a first application means (sample application pad) 130 and a second application means (detector application pad) 131. The first application means 130 and the second application means 131 are not in operable contact until the first opposable component 121 and the second opposable component 122 are brought into opposition. When the first opposable component 121 and the second opposable component 122 are brought into opposition, by closing the hinge 133, the first application means 130 and the second application means 131 are bridged by the conducting means 126. Thus, the path of fluid flow is from the first application means 130 through the conducting means 126, then through the second application means 131, and then into the chromatographic medium 123. The second opposable component 122 contains a window 132 to allow viewing of the chromatographic medium 123.

### c. Device With Pad for Labelled Specific Binding Partner on Same Opposable Component as Chromatographic Medium

Yet another embodiment of a chromatographic assay device according to the present invention is a two-component device incorporating a pad for a labelled specific binding partner on the same opposable component as the chromatographic medium. In this device, the sample application means is located on the other opposable component.

This embodiment of a chromatographic assay device according to the present invention is depicted in Figure 6A. The chromatographic assay device 140 has a first opposable component 142 and a second opposable component 144. The first opposable component 142 has a chromatographic medium 146 having a first end 148 and a second end 150. The chromatographic medium has a detection zone 162 and a control zone 164. The first opposable component 142 also has a conducting means 152 in operable contact with the first end 148 of the chromatographic medium 146, and an absorbing means 154 in operable contact with the second end 150 of the chromatographic medium 146. The first opposable component 142 also has a detector application pad 156 in direct contact with the conducting means 152 and positioned such that it is in indirect contact with the first end 148 of the chromatographic medium 146. The second opposable component 144 has a sample application pad 158. The first opposable component 142 and the second opposable component 144 are joined by a hinge 160. When the first opposable component 142 and the second opposable component 144 are brought into opposition, the sample application pad 158 is brought into contact with the detector application pad 156. The second opposable component 144 contains a window 166 to allow viewing of the chromatographic medium 146.

A side view of the device 140 is depicted in Figure 6B. The section shown in Figure 6B is taken from the view of Figure 6A between the chromatographic medium 146 and the hinge 160 looking toward the edge opposite the hinge 160. Figure 6B shows the first opposable component 142 and second opposable component 144 in opposition. The sample application pad 158 is shown in contact with the detector application pad 156. The detector application pad 156 is in contact with the conducting means 152, which is in turn in contact with the first end 148 of the chromatographic medium 146. The detection zone 162 and control zone 164 of the chromatographic medium 146 are shown. The second end 150 of the chromatographic medium 146, nearer the control zone 164, is in contact with the absorbing means 154.

Bringing the first and second opposable components into opposition causes the sample application pad to apply the sample to be tested to the detector application pad and thus to the first end of the chromatographic medium though the conducting means.

Preferably, the detector application pad contains a first specific binding partner to the analyte in a form that can be resolubilized by addition of an aqueous liquid to the detector application pad, and the first specific binding partner is labelled with a detectable label. Preferably, the chromatographic medium further comprises a detection zone substantially smaller in area than the chromatographic medium, as described above. In this arrangement, a ternary complex comprising the first (labelled) specific binding partner, the analyte, and the second specific binding partner forms at the detection zone if analyte is present in the sample. This ternary complex is what is detected or determined.

Preferably, the contents of the sample application pad after a sample is applied thereto comprises an aqueous liquid, and the aqueous liquid applied to the detector application pad comprises the contents of the sample application pad. In this arrangement, there is no additional liquid needed to resolubilize the labelled specific binding partner.

In use, a sample is applied to the sample application pad, and the first and second opposable components are brought into opposition. This applies the sample to the detector application pad, resolubilizing the labelled specific binding partner. The sample and the labelled specific binding partner then flow through the conducting means and into the chromatographic medium for detection and/or determination as described above.

A variation of this device omits the conducting means between the detector application pad and the chromatographic medium, so that the detector application pad is in direct contact with the first end of the chromatographic medium. In this variation, when the first and second opposable components are brought into opposition, the detector application pad and the sample application pad are in contact except for the region of the detector application pad directly adjacent to the first end of the chromatographic medium. There is a slight gap or offset at that region of the detector application pad, so that sample cannot flow directly from the sample application pad to the detector application pad. This gap or offset is typically from about 0.5 mm to about 2 mm, more typically from about 0.5 mm to about 1 mm.

This variation is particularly suitable for the detection of fecal occult blood by use of a labelled anti-hemoglobin antibody, and can detect concentrations of hemoglobin corresponding to as much as 17 ml of blood per 100 g feces (13 mg hemoglobin per gram of feces), without the occurrence of false negatives due to a high dose "hook" effect.

This variation is depicted in Figure 7A. The chromatographic assay device 180 has a first opposable component 182 and a second opposable component 184. The first opposable component 182 has a chromatographic medium 186 having a first end 188 and a second end 190. The chromatographic medium has a detection zone 202 and a control zone 204. The first opposable component 182 also has an absorbing means 192 in operable contact with the second end 190 of the chromatographic medium 186. The first opposable component 182 also has a detector application pad 194 in direct contact with the first end 188 of the chromatographic medium 186. The second opposable component 184 has a sample application pad 196. The first opposable component 182 and the second opposable component 184 are joined by a hinge 198. When the first opposable component 182 and the second opposable component 184 are brought into opposition, the sample application pad 196 is brought into contact with the detector application pad 194, except for a narrow gap or offset 200 at the end of the detector application pad 194 in contact with the first end 188 of the chromatographic medium 186. The second opposable component 184 has a window 206 to allow viewing of the chromatographic medium 186.

A side view of the device 180 of Figure 7A is depicted in Figure 7B. The section shown in Figure 7B is taken from the view of Figure 7A between the chromatographic medium 186 and the hinge 198 looking toward the edge opposite the hinge 190. Figure 7B shows the first opposable component 182 and second opposable component 184 in opposition, with the hinge 198 in closed position. The sample application pad 196 is shown in contact with the detector application pad 194, except for a small gap 200 at the end of the detector application pad 194 nearest the chromatographic medium 186. This gap 200 prevents sample applied to the sample application pad 196 from flowing directly into the chromatographic medium 186. The detector application pad 194 is in direct contact with the first end 188 of the chromatographic medium 186. The detection zone 202 and control zone 204 of the chromatographic medium 186 are shown. The second end 190 of the chromatographic medium 186, nearer the control zone 204, is in contact with the absorbing means 192.

### C. Three-Component Device

Another embodiment of a chromatographic assay device according to the present invention is a three-component assay device utilizing bi-directional chromatography.

This embodiment of the three-component assay device is shown in Figure llA. The assay device 400 has a first opposable component 402, a second opposable component 404, and a third opposable component 406. The second opposable component 404 is hingedly attached to one side of the first opposable component 402 by a first hinge 408; the third opposable component 406 is hingedly attached to the opposite side of the first opposable component 402 by a second hinge 410. The first opposable component 402 has a chromatographic medium 412 having a first end 414 and a second end 416. The chromatographic medium 412 has a detection zone 418 and a control zone 420. In operable contact with the first end 414 of the chromatographic medium 412 is a first conducting means 422 and in operable contact with the second end 416 of the chromatographic medium 412 is a second conducting means 424. The second opposable component 404 comprises a first absorbing means 426 and a first application means 428, intended for application of the sample. A first aperture 430 is cut in the second opposable component 404 to allow viewing of at least a portion of the chromatographic medium 412. The first aperture 430 is between the first absorbing means 426 and the first application means 428. The third opposable component 406 comprises a second application means 432 intended for a labelled secondary specific binding partner and a second absorbing means 434. A second aperture 436 is cut in the third opposable component 406 to allow viewing of at least a portion of the chromatographic medium 412. The second aperture 436 is between the second application means 432 and the second absorbing means 434. When the device 400 is closed, with the second opposable component 404 folded over the first opposable component 402 and the third opposable component 406 folded over the first opposable component 402, at least a portion of the chromatographic medium 412 is visible through the first aperture 430 and the second aperture 436 (Fig. 11B).

In this device, bringing the first and second opposable components into opposition causes the first absorbing means to come into operable contact with the second conducting means and causes the first application means to come into operable contact with the first conducting means to apply fluid to the chromatography medium, so that a first liquid applied to the first application means is drawn through at least a portion of the chromatographic medium.

The first and second opposable components are then withdrawn from opposition and the first and third opposable components are brought into opposition. This causes the second absorbing means to come into operable contact with the first conducting means to withdraw fluid from the chromatography medium and causes the second application means to come into operable contact with the second conducting means to apply fluid to the chromatography medium. This causes a reversal of flow so that a second liquid applied to the second application means is drawn through at least a portion of the chromatographic medium through which the first liquid has been drawn in the direction opposite to the direction in which the first liquid was drawn through the chromatographic medium. The first, second, and third opposable components are in such a configuration that, when the third opposable component is brought into opposition with the first opposable component, the second opposable component can be folded over the first and third opposable components to form a cover. Both the second and third opposable components contain apertures that allow viewing of at least a portion of the chromatographic medium.

In the performance of an assay using this device, firm pressure between the second absorbing means and the first conducting means is important to assure that non-specific IgG is withdrawn from the medium before the advancing front of anti-IgG label. If mixing occurs, the non-specific IgG neutralizes the conjugate leaving less/none available for labeling the specifically captured IgG. The third opposable component of the device helps keep consistent pressure applied to reliably effect this function. This is one of the advantages of assay devices according to the present invention.

Typically, the first application means contains a first specific binding partner for the analyte in a form that can be resolubilized by the application of an aqueous sample to the first application means. The first specific binding partner can be directly labelled with a detectable label. Alternatively, indirect labelling of the first specific binding partner can be used. Indirect labeling is particularly useful for testing for Giardia or other antigens for which commercially available antibodies are directly labelled only with difficulty. When the first specific binding partner is not directly labelled, the second application means preferably contains a labelled secondary specific binding partner for the first specific binding partner, as discussed below in Section II.

### II. ANALYTES AND ANTIBODIES FOR USE WITH THE ASSAY DEVICE

The analytes suitable for detection with an assay device according to the present invention include antigens, haptens, and antibodies. Antigens detectable with the device include hemoglobin, Streptococcus A and B antigens, antigens specific for the protozoan parasite Giardia, and viral antigens, including antigens specific for HIV and the Australia antigen specific for hepatitis. Antibodies that can be assayed include antibodies to bacteria such as Helicobacter pylori and to viruses, including HIV.

If the analyte is a hapten or antigen, the first and second specific binding partners are preferably antibodies. In many applications, it is preferable that the first and second specific binding partners are antibodies to different epitopes on the analyte, but this is not required. The antibodies can be polyclonal or monoclonal, and can be IgG, IgM or IgA. In many applications, polyclonal antibodies are preferred, as their natural variability may allow more accurate detection in systems where antigenic polymorphisms exist or may exist.

When the analyte is a hapten, it is strongly preferred that the first and second specific binding partners be antibodies to different epitopes; otherwise, there may be an undesirable competition reaction set up that may interfere with binding of the complex of the labelled specific binding partner and the analyte to the immobilized second specific binding partner.

Where the analyte is an antibody, the first specific binding partner is typically a labelled antibody that binds to the analyte on the basis of species, class, or subclass (isotype) specificity. It is highly preferred that the first specific binding partner to an antibody analyte binds to the constant region of the antibody analyte, in order to prevent interference. When the analyte is antibody, the second specific binding partner is preferably an antigen or hapten for which the antibody analyte is specific.

In some applications, it is desirable to employ indirect labelling. For example, in testing for Giardia antigen, an IgM antibody can be used that may be difficult to label directly. In that case, a secondary specific binding partner specific for the mobile first specific binding partner can be labelled. Typically, the labelled secondary specific binding partner binds to the antibody that is the first specific binding partner on the basis of species, class, or subclass specificity.

As an alternative to the use of a secondary specific binding partner, the first specific binding partner can be conjugated to biotin and an avidin-conjugated label can be used.

These relationships between analytes, specific binding partners, and labels are summarized in Table 1 below.

**TABLE I**

| SCHEMES OF BINDING | | | | |
|---|---|---|---|---|
| ANALYTE | 1ST SBP (MOBILE) | 2ND SBP (FIXED) | SECONDARY SBP | COMPLEX FORMED |
| Ag | Ab₁* | Ab₂ | --- | Ab₂-Ag-Ab₁* |
| H | Ab_{1*} | Ab₂ | --- | Ab₂-H-Ab₁*⁽¹⁾ |
| Ab | Ab_{c}* | Ag | --- | Ag-Ab-Ab_{c}* |
| Ag | Ab₁ | Ab₂ | Ab_{c}* | Ab₂-Ag-Ab₁-Ab_{c}* |
| Ab | Ab_{c1} | Ag | Ab_{c2}* | Ag-Ab-Ab_{c1}-Ab_{c2}* |
| Ag | Ab₁-Bi | Ab₂ | Av-L | Ab₂-Ag-Ab₁-Bi-Av-L |

| | | | | |
|---|---|---|---|---|
| Ag = Antigen H = Hapten Ab₁ = 1st Antibody Ab₂ = 2nd Antibody Ab_{c}, Ab_{c1}, Ab_{c2} = Antibody specific for another antibody Bi = Biotin Av = Avidin L = Label *Indicates labelled component | | | | |
| ⁽¹⁾ Ab₂ and Ab₁* preferred to bind to different epitopes | | | | |

Chromatographic assay devices according to the present invention can readily be adapted for the performance of competitive immunoassays for analytes that bind antibodies in a monovalent manner, such as drugs and other haptens. In such competitive immunoassays, the labeled conjugate is an antibody-label conjugate and the detection zone comprises immobilized analyte analogue. The antibody-label conjugate is present along with a quantity of unlabeled antibody sufficient to prevent binding of the antibody-label conjugate to the immobilized analyte analogue in the absence of analyte in the test sample.

### III. TEST KITS

Another aspect of the present invention is test kits that can be used to detect particular analytes. A test kit comprises:
(1) a chromatographic assay device according to the present invention;
(2) any necessary reagents required to treat or extract the sample; and
(3) optionally, if the assay device does not incorporate a labelled specific binding partner to the analyte in a form that can be resolubilized, the required specific binding partner.

The components required in (2) and (3) are packaged separately and can be in liquid or solid form (freeze-dried, crystallized, precipitated, or aggregated). If the latter, they are resolubilized by the user, typically with distilled or purified water, with physiological saline, or a buffer solution.

Still other variations of test devices according to the present invention are possible, For example, any of the two-component devices described can have a cover hingedly attached to one of the opposable components. This cover can have an aperture cut therein to allow viewing of at least a portion of the chromatographic medium.

The invention is illustrated by the following Examples. The Examples are for illustrative purposes only and are not to be construed as limiting the scope of the invention.

### EXAMPLES

### Example 1

### Construction of Device for Detecting Streptococcal Antigen

A device was constructed for detecting Streptococcus A antigen using labelled antibody to Streptococcus A antigen.
The device was constructed essentially as depicted in Figure 16.

Figure 16 shows a chromatographic assay device 700 according to the present invention with a first opposable component 702, a second opposable component 704 hingedly attached to the first opposable component 702, and a cover 706 hingedly attached to the second opposable component 704. The first opposable component 702 includes a chromatographic medium 708. The second opposable component 704 includes a teardrop-shaped well 710 held in place by a ribbon 712. The first opposable component 702 contains a first window 714 and the cover contains a second window 716.

The opposable components were made of a hard, impervious plastic such as Lexan®. The first and second opposable components, as well as the cover, each were about 3" in length; the first opposable component was about 2.25" in width, while the second opposable component and the cover was each about 2.375" in width. The second opposable component was lined with foam rubber, into which a teardrop-shaped well was cut to accept a swab or other sampling device. The swab was held in place with a ribbon separately inserted into the second opposable component across the well.

The chromatographic medium was a nitrocellulose strip 8 µm thick and 0.5" in length, (MSI, Westborough, Mass.), affixed to the plastic backing by means of double-sided tape (3M, Minneapolis, Minnesota). The conducting means and absorbing means were cellulose strips (Ahlstrom Filtration, Holly Springs, Pennsylvania), 17/32" in length for the absorbing means, which was Ahlstrom Grade 939, and 0.25" in length for the conducting means, which was Ahlstrom Grade 1281. The detector application pad was also Ahlstrom Grade 1281, and was 0.375" wide. The detector application pad overlapped slightly with the conducting means, which in turn overlapped slightly with the chromatographic medium at its first end. The chromatographic medium overlapped slightly at its second end with the absorbing means.

The required reagents were first incorporated in the chromatographic medium and the detector application pad, after which the device was assembled using double-sided tape to hold the components to the backing.

The detection zone comprised rabbit anti-Streptococcus A antibody at 2 mg/ml in 0.001 mole/l phosphate buffered saline, pH 7.2. The control zone comprised goat anti-rabbit IgG at a similar concentration in the same buffer. The antibody solutions were applied to the appropriate regions of the chromatographic medium and dried at 100°F in a low humidity environment. The chromatographic medium was wet in excess blocking solution (Blocking Reagent for ELISA, Boehringer Mannheim, Mannheim, Germany, diluted 1:10 with distilled water containing 0.2% Tween 20) and again dried at 100°F.

The detector application pad contained rabbit anti-Streptococcus antibody labelled with 40-nm colloidal gold particles. To apply the labelled antibody to the detector application pad, the labelled antibody was diluted 1:1.5 with DBN (1.5 mole/l Tris-HCl, pH 7.4, 1% (v/v) Tween 20, 0.4% (v/v) Brij 35, 0.02% (w/v) sodium azide, 3 mg/ml rabbit IgG). Per test, 15 µl of diluted labelled antibody was added to the detector application pad. The detector application pad was dried for 30 minutes at 100°F.

### Example 2

### Detection of Streptococcal Antigen Using Device of Example 1

The device of Example 1 was used to detect Streptococcus A antigen. A woven dacron swab to which varying quantities of Streptococcus type A bacteria had been added was inserted into the sample well. Three drops of Extraction Reagent A (0.25% acetic acid, 5% Tween 20), and three drops of Extraction Reagent B (2 mole/l sodium nitrite, 5% Tween 20) were added to the swab, mixed by gently rotating the swab, and incubated for one minute. The device was then closed, so that the first and second opposable components were brought into contact, and the cover was then folded over the first opposable component. The result was read after an incubation period of from 2 minutes to 5 minutes. The development of a pink-red band in the detection zone of the chromatographic medium indicated the detection of Streptococcus A antigen.

The device of Example 1 could detect 1x10⁵ Streptococcus A organisms after a 2-minute incubation, and could detect 5x10⁴ Streptococcus A organisms after a 5-minute incubation. For a comparison, the Concise™ immunoassay of Hybritech (La Jolla, California) could detect 1x10⁵ Streptococcus A organisms only after a 5-minute incubation, and could not detect 5x10⁴ Streptococcus A organisms even after a 20-minute incubation. Similarly, the Smart™ immunoassay of New Horizons could detect 1x10⁵ Streptococcus A organisms only after a 7-minute incubation, and gave an equivocal result with 5x10⁴ Streptococcus A organisms after a 7-minute incubation.

### Example 3

### Device for Detecting Hemoglobin in Fecal Occult Blood

### (Prospective Example)

An assay device for the detection of hemoglobin in fecal occult blood is constructed according to Figures 1A and 1B, incorporating the optional conducting means at the first end of the chromatographic medium. A labeled specific binding partner is applied to the sample application pad in resolubilizable form. The labeled specific binding partner is goat anti-human hemoglobin antibody labeled with colloidal gold. A fecal sample of 60 µl is applied to the sample application pad and allowed to mix with conjugate. The device is closed and the combination of the fecal sample and reconstituted antibody contacts the conducting means and moves through the chromatographic medium. Chromatography is allowed to proceed for a period of about 1 minute to about 5 minutes. The chromatographic medium contains a detection zone of immobilized anti-human Hb antibody, and a control zone of immobilized rabbit anti-goat IgG antibody. Color appearing at both the detection zone and the control zone indicates a positive result, i.e., the presence of occult blood in the fecal sample. Color appearing at the control zone, but not at the detection zone, indicates the absence of occult blood and the correct performance of the test.

This device is capable of detecting hemoglobin in fecal occult blood in a concentration range of from about 0.2 ml blood/100 g feces to about 17 ml blood/100 g feces. This device is free from interference caused by peroxidase and dietary (non-human) hemoglobin.

### ADVANTAGES OF THE INVENTION

Chromatographic assay devices according to the present invention allow the rapid and accurate detection of clinically important analytes, such as Streptococcus A and B antigen, hemoglobin for the determination of fecal occult blood, and antibody to Helicobacter pylori. The construction of the devices allows more even application of the samples to the chromatographic medium, and reduces interference that might otherwise be introduced by particulates or colored samples. The use of colloidal metal labels in a resolubilizible form provides extremely rapid kinetics of labeling and allows substantially complete formation of binary analyte-label complexes before the sample is applied to the chromatographic medium. This aids in the separation of contaminants and improves the performance of the assay. Additionally, the construction and arrangement of the housing of the device aids in the performance of the assay by assuring the withdrawal of excess immunoglobulin-containing sample that could otherwise create interference.

Extraction of biological samples such as blood, sputum, or feces can be performed directly in the devices, reducing the quantity of contaminated material that must be disposed and reducing the likelihood of accidental infection of physicians, technicians, or the public by such contaminated material. Additionally, the devices are capable of performing bi-directional chromatography to further increase accuracy and reduce interference. Test methods using devices according to the present invention have a wide dynamic range and are substantially free from false negatives that may occur in other test methods at high concentrations of analyte.

Although the present invention has been described with considerable detail, with reference to certain preferred versions thereof, other versions and embodiments are possible. These versions include other arrangements of two- or three-component devices that operate by the basic principles described herein and utilize any of: (a) in situ extraction of samples; (b) resolubilization of a labeled specific binding partner and rapid binding to analyte; and (c) arrangement of the chromatographic medium and absorbing means to remove excess sample that could otherwise create interference. These versions include assay devices adapted for competitive immunoassays. Therefore, the scope of the invention is determined by the following claims.

## Claims

1. A chromatographic assay device for detection and/or determination of at least one analyte comprising at least first and second opposable components, one of the first and second opposable components including a chromatographic medium, and one of the first and second opposable components including a sample preparation means, the opposable component including the chromatographic medium and the opposable component including the sample preparation means being different opposable components, wherein the first and second opposable components can be brought into opposition so as to cause a sample liquid from the sample preparation means to be applied to the component including the chromatographic medium so as to contact said chromatographic medium.

2. The chromatographic assay device of claim 1 wherein the device (10) comprises:
(a) a first opposable component (14) including a chromatographic medium (18) having first and second ends (19, 20); and
(b) a second opposable component (12) including a sample preparation means (16) adapted to receive a sample to be assayed; wherein the first and second opposable components can be brought into opposition so as to cause the sample preparation means to apply the sample to be tested to the chromatographic medium.

3. The chromatographic assay device of claim 1 wherein the device (400) comprises:
(a) a first opposable component (402) including:
(i) a chromatographic medium (412) having first and second ends (414, 416);
(ii) a first conducting means (422) in operable contact with the first end of the chromatographic medium; and
(iii) a second conducting means (424) inoperable contact with the second end of the chromatographic medium;
(b) a second opposable component (404) hingedly attached to the first opposable component and including:
(i) a first absorbing means (426); and
(ii) a first application means (428) separated from the first absorbing means and including therein a sample preparation means adapted to receive a sample to be assayed;
wherein bringing the first and second opposable components into opposition causes the first absorbing means to come into operable contact with the second conducting means to withdraw fluid from the chromatographic medium, and causes the first application means to come into operable contact with the first conducting means to apply fluid to the chromatographic medium, so that a first liquid applied to the first application means is drawn through at least a portion of the chromatographic medium; and
(c) a third opposable component (406) hingedly attached to the first opposable component and including:
(i) a second absorbing means (434); and
(ii) a second application means (432) separated from the second absorbing means;
wherein bringing the first and third opposable components into opposition causes the second absorbing mean to come into operable contact with the first conducting means to withdraw fluid from the chromatographic medium and causes the second application means to come into operable contact with the second conducting means to apply fluid to the chromatographic medium so that a second liquid applied to the second application means is drawn through at least a portion of the chromatographic medium overlapping the portion of the chromatographic medium through which the first liquid is drawn; the first, second and third opposable components being in such a configuration that when the third opposable component is brought into opposition with the first opposable component, the second opposable component can be folded over the first and third opposable components to form a cover.

4. The chromatographic assay device of claim 1 wherein the device (90) comprises:
(a) a first opposable component (92) including:
(i) a chromatographic medium (96) having first and second ends (98, 100);
(ii) a conducting means (102) in operable contact with the first end of the chromatographic medium; and
(iii) an absorbing means (104) in operable contact with the second end of the chromatographic medium; and
(b) a second opposable component (94) including:
(i) a first application means (110) including a sample preparation means adapted to receive a sample to be assayed; and
(ii) a second application means (112), the first and second application means being positioned on the second opposable component such that they are not in operable contact when the first and second opposable components are not in opposition;
wherein bringing the first and second opposable components into opposition places the conducting means in operable contact with the first application means and places the conducting means in operable contact with the second application means, thereby placing the first and second application means in operable contact with each other.

5. The chromatographic assay device of claim 1 wherein the device (140) comprises:
(a) a first opposable component (142) including:
(i) a chromatographic medium (146) having first and second ends (148, 150);
(ii) a conducting means (152) in operable contact with the first end of the chromatographic medium;
(iii) an absorbing means (154) in operable contact with the second end of the chromatographic medium; and
(iv) a detector application pad (156) in direct contact with the conducting means and positioned such that it is in indirect contact with the first end of the chromatographic medium; and
(b) a second opposable component (144) including a sample preparation means adapted to receive a sample to be assayed including a sample preparation pad (158); whereby bringing the first and second opposable components into opposition causes the sample application pad to apply the sample to be tested to the detector application pad and thus the first end of the chromatographic medium through the conducting means.

6. The chromatographic assay device of claim 1 wherein the device (180) comprises:
(a) a first opposable component (182) including:
(i) a chromatographic medium (186) having first and second ends (188, 190);
(ii) an absorbing means (192) in operable contact with the second end of the chromatographic medium; and
(iii) a detector application pad (194) in direct contact with the first end of the chromatographic medium; and
(b) a second opposable component including a sample preparation means adapted to receive a sample to be assayed including a sample application pad (196);
wherein the first and second opposable components are brought into opposition, the detector application pad and the sample application pad are in contact except for the region of the detector application pad directly adjacent to the first end of the chromatographic medium, and whereby bringing the first and second opposable components into opposition causes the sample application pad to apply to the sample to be tested to the detector application pad and thus to the first end of the chromatographic medium.

7. The chromatographic assay device of claim 1, wherein the device (120) comprises:
(a) a first opposable component (121) including:
(i) a chromatographic medium (123) having first and second ends (124, 125);
(ii) a conducting means (126) positioned such that it is not in operable contact with the first end of the chromatographic medium when the first opposable component and second opposable component are not in opposition; and
(iii) an absorbing means (127) in operable contact with the second end of the chromatographic medium; and
(b) a second opposable component (122) comprising:
(i) a first application means (130) including a sample preparation means adapted to receive a sample to be assayed; and
(ii) a second application means (131);
the first and second application means being positioned on the second opposable component such that they are not in operable contact when the first and second opposable components are not in opposition; wherein bringing the first and second opposable components into opposition places the conducting means into operable contact with the first application means, places the conducting means in operable contact with the second application means, and places the second application means in operable contact with the first end of the chromatographic medium, thereby placing the first and second application means in operable contact with each other to apply the contents of the first and second application means to the chromatographic medium.

8. The chromatographic assay device of claim 2, 3, 4, 5, 6 or 7 wherein the chromatographic medium further includes a detection zone (20; 106; 128; 162; 202; 418) substantially smaller in area than the chromatographic medium.

9. The chromatographic assay device of claim 8 wherein the detection zone includes a specific binding partner for the analyte immobilized to the chromatographic medium.

10. The chromatographic assay device of claim 8 wherein the chromatographic medium further includes a central zone (22; 108; 129; 164; 204; 420) substantially smaller than the chromatographic medium and separated from the detection zone.

11. The chromatographic assay device of claim 10 wherein the control zone includes analyte immobilized thereto.

12. The chromatographic assay device of claim 2 wherein the device further comprises at least one of:
(i) a conducting means (35) in operable contact with the first end of the chromatographic medium and
(ii) an absorbing means (36) in operable contact with the second end of the chromatographic medium.

13. The chromatographic assay device of claim 2 wherein the sample preparation means further contains a specific binding partner for the analyte labeled with a detectable label in a form that can be resolubilized by the addition of an aqueous liquid to the sample preparation means.

14. The chromatographic assay device of claim 2 wherein the sample preparation means contains at least one reagent for treatment of the sample before the sample is applied to the chromatographic medium.

15. The chromatographic assay device of claim 2 wherein the first and second opposable components each further include engaging means (26, 28) for securing the first and second opposable components in opposition.

16. The chromatographic assay device of claim 7 wherein the sample preparation means includes a sample application pad (428) containing at least one reagent for treatment of the sample before the sample is applied to the chromatographic medium.

17. The chromatographic assay device of claim 3 wherein the first application means (428) contains a first specific binding partner for the analyte in a form that can be resolubilized by the addition of an aqueous sample to the application means.

18. The chromatographic assay device of claim 3 wherein the second liquid comprises a specific binding partner to the analyte and the chromatographic medium further comprises a detection zone (418) containing a secondary specific binding partner, the secondary specific binding partner being specific for the first specific binding partner.

19. The chromatographic assay device of claim 4 wherein the sample preparation means includes a sample application pad (110) and the second application means includes a detector application pad (112), to which detecting reagent can be applied, whereby, when the first and second opposable components are brought into opposition, the contents of the sample application pad and the detector application pad are applied to the conducting means (102), the detector application pad containing a first specific binding partner to the analyte in a form that can be resolubilized by addition of an aqueous liquid to the detector application pad, the first specific binding partner being labeled with a detectable label, and the chromatographic medium further comprises a detection zone (106) substantially smaller in area than the chromatographic medium, the detection zone containing a second specific binding partner to the analyte immobilized thereto, such that a ternary complex comprising the first specific binding partner, the analyte, and the second specific binding partner forms at the detection zone if analyte is present in the sample.

20. The chromatographic assay device of claim 5 or 6 wherein the detector application pad (156; 194) contains a first specific binding partner to the analyte in a form that can be resolubilized by addition of an aqueous liquid to the detector application pad, the first specific binding partner being labeled with a detectable label, and the chromatographic medium further comprises a detection zone (162; 202) substantially smaller in area than the chromatographic medium, the detection zone containing a second specific binding partner to the analyte immobilized thereto, such that a ternary complex comprising the first specific binding partner, the analyte, and the second specific binding partner forms at the detection zone if analyte is present in the sample.

21. The chromatographic assay device of claim 4 wherein the sample preparation means includes a sample application pad (130) and the second application means includes a detector application pad (131), to which detecting reagent can be applied, whereby, when the first and second opposable components are brought into opposition, the contents of the sample application pad and the detector application pad are applied to the chromatographic medium, the detector application pad containing a first specific binding partner to the analyte in a form that can be resolubilized by addition of an aqueous liquid to the detector application pad, the first specific binding partner being labeled with a detectable label, and the chromatographic medium further comprises a detection zone (128) substantially smaller in area than the chromatographic medium, the detection zone containing a second specific binding partner to the analyte immobilized thereto, such that a ternary complex comprising the first specific binding partner, the analyte, and the second specific binding partner forms at the detection zone if analyte is present in the sample.

22. The chromatographic assay device of claim 13, 19, 20 or 21 wherein the detectable label is a visually detectable label.

23. The chromatographic assay device of claim 4 wherein the analyte is human hemoglobin and the specific binding partner is an anti-human hemoglobin antibody.

24. A test kit for the detection and/or determination of an analyte comprising:
(a) a chromatographic assay device of claim 2, 4, 5, 6 or 7; and
(b) a specific binding partner for the analyte labeled with a detectable label.

25. A test kit for the detection and/or determination of an analyte comprising:
(a) the chromatographic assay device of claim 3; and
(b) a specific binding partner for the first specific binding partner to the analyte labeled with a detectable label.

26. The test kit of claim 24 or 25 wherein the detectable label is a visually detectable label.

27. A test kit for the detection and/or determination of an analyte, comprising:
(a) the chromatographic assay device of claims 4, 5, 6 or 7; and
(b) an aqueous liquid for resolubilizing the specific binding partner for the analyte labeled with a detectable label, to be applied to the detector application pad.

28. The test kit of claim 24, 25, 26 or 27, wherein the analyte to be detected is human hemoglobin.

## Patentansprüche

1. Chromatographische Untersuchungsvorrichtung zur Erfassung und/oder Bestimmung zumindest eines Analyten mit zumindest ersten und zweiten gegenüberstellbaren Bestandteilen, wobei einer der ersten und zweiten gegenüberstellbaren Bestandteile ein chromatographisches Medium einschließt, und einer der ersten und zweiten gegenüberstellbaren Bestandteile eine Probenvorbereitungseinrichtung einschließt, wobei der gegenüberstellbare Bestandteil, der das chromatographische Medium einschließt und der gegenüberstellbare Bestandteil, der die Probenvorbereitungseinrichtung einschließt, unterschiedliche gegenüberstellbare Bestandteile sind, wobei die ersten und zweiten gegenüberstellbaren Bestandteile in Gegenüberstellung gebracht werden können, um zu bewirken, daß eine Probenflüssigkeit von der Probenvorbereitungseinrichtung auf den Bestandteil aufgebracht wird, der das chromatographische Medium einschließt, um mit dem chromatographischen Medium in Berührung zu kommen.

2. Chromatographische Untersuchungsvorrichtung nach Anspruch 1,
bei der die Vorrichtung (10) folgendes umfaßt:
(a) einen ersten gegenüberstellbaren Bestandteil (14), der ein chromatographisches Medium (18) mit ersten und zweiten Enden (19,20) einschließt; und
(b) einen zweiten gegenüberstellbaren Bestandteil (12), der eine Probenvorbereitungseinrichtung (16) einschließt, die geeignet ist, eine zu untersuchende Probe aufzunehmen; wobei die ersten und zweiten gegenüberstellbaren Bestandteile in Gegenüberstellung gebracht werden können, um zu bewirken, daß die Probenvorbereitungseinrichtung die zu testende Probe auf das chromatographische Medium aufbringt.

3. Chromatographische Untersuchungsvorrichtung nach Anspruch 1,
bei der die Vorrichtung (400) folgendes umfaßt:
(a) einen ersten gegenüberstellbaren Bestandteil (402), einschließlich:
(i) eines chromatographischen Mediums (412) mit ersten und zweiten Enden (414,416);
(ii) einer ersten Leitungseinrichtung (422) in Betriebsberührung mit dem ersten Ende des chromatographischen Mediums; und
(iii) einer zweiten Leitungseinrichtung (424) in Betriebsberührung mit dem zweiten Ende des chromatographischen Mediums;
(b) einen zweiten gegenüberstellbaren Bestandteil (404), der gelenkig am ersten gegenüberstellbaren Bestandteil angebracht ist und folgendes einschließt:
(i) eine erste Absorptionseinrichtung (426); und
(ii) eine erste Aufbringungseinrichtung (428), die von der ersten Absorptionseinrichtung getrennt ist und eine Probenvorbereitungseinrichtung einschließt, die zur Aufnahme einer zu untersuchenden Probe ausgebildet ist;
wobei das In-Gegenüberstellung-Bringen der ersten und zweiten gegenüberstellbaren Bestandteile bewirkt, daß die erste Absorptionseinrichtung mit der zweiten Leitungseinrichtung in Betriebsberührung kommt, um Strömungsmittel vom chromatographischen Medium abzuziehen, und bewirkt, daß die erste Aufbringungseinrichtung mit der ersten Leitungseinrichtung in Betriebsberührung kommt, um Strömungsmittel auf das chromatographische Medium aufzubringen, so daß eine erste Flüssigkeit, die auf die erste Aufbringungseinrichtung aufgebracht wird, durch zumindest einen Teil des chromatographischen Mediums gezogen wird; und
(c) einen dritten gegenüberstellbaren Bestandteil (406), der gelenkig am ersten gegenüberstellbaren Bestandteil angebracht ist und folgendes einschließt:
(i) eine zweite Absorptionseinrichtung (434); und
(ii) eine zweite Aufbringungseinrichtung (432), die von der zweiten Absorptionseinrichtung getrennt ist;
wobei ein In-Gegenüberstellung-Bringen der ersten und zweiten gegenüberstellbaren Bestandteile bewirkt, daß die zweite Absorptionseinrichtung mit der ersten Leitungseinrichtung in Betriebsberührung kommt, um Strömungsmittel vom chromatographischen Medium abzuziehen und bewirkt, daß die zweite Aufbringungseinrichtung mit der zweiten Leitungseinrichtung in Betriebsberührung kommt, um Strömungsmittel auf das chromatographische Medium aufzubringen, so daß eine zweite, auf die zweite Aufbringungseinrichtung aufgebrachte Flüssigkeit durch zumindest einen Teil des chromatographischen Mediums gezogen wird, der den Teil des chromatographischen Mediums überlappt, durch den die erste Flüssigkeit gezogen wird; wobei die ersten, zweiten und dritten gegenüberstellbaren Bestandteile derartig ausgebildet sind, daß, wenn der dritte gegenüberstellbare Bestandteil mit dem ersten gegenüberstellbaren Bestandteil in Gegenüberstellung gebracht wird, der zweite gegenüberstellbare Bestandteil über die ersten und dritten gegenüberstellbaren Bestandteile faltbar ist, um eine Abdeckung zu bilden.

4. Chromatographische Untersuchungsvorrichtung nach Anspruch 1,
bei der die Vorrichtung (90) folgendes umfaßt:
(a) einen ersten gegenüberstellbaren Bestandteil (92), der folgendes einschließt:
(i) ein chromatographisches Medium (96) mit ersten und zweiten Enden (98,100);
(ii) eine Leitungseinrichtung (102) in Betriebsberührung mit dem ersten Ende des chromatographischen Mediums; und
(iii) eine Absorptionseinrichtung (104) in Betriebsberührung mit dem zweiten Ende des chromatographischen Mediums; und
(b) einen zweiten gegenüberstellbaren Bestandteil (94), der folgendes einschließt:
(i) eine erste Aufbringungseinrichtung (110), einschließlich einer Probenvorbereitungseinrichtung, die zur Aufnahme einer zu untersuchenden Probe ausgebildet ist; und
(ii) eine zweite Aufbringungseinrichtung (112), wobei die ersten und zweiten Aufbringungseinrichtungen auf dem zweiten gegenüberstellbaren Bestandteil derartig angeordnet sind, daß sie nicht in Betriebsberührung sind, wenn die ersten und zweiten gegenüberstellbaren Bestandteile nicht in Gegenüberstellung sind;
wobei das In-Gegenüberstellung-Bringen der ersten und zweiten gegenüberstellbaren Bestandteile die Leitungseinrichtung in Betriebsberührung mit der ersten Aufbringungseinrichtung bringt und die Leitungseinrichtung in Betriebsberührung mit der zweiten Aufbringungseinrichtung bringt, wodurch die ersten und zweiten Aufbringungseinrichtungen miteinander in Betriebsberührung gebracht werden.

5. Chromatographische Untersuchungsvorrichtung nach Anspruch 1,
bei der die Vorrichtung (140) folgendes umfaßt:
(a) einen ersten gegenüberstellbaren Bestandteil (142), der folgendes einschließt:
(i) ein chromatographisches Medium (146) mit ersten und zweiten Enden (148,150);
(ii) eine Leitungseinrichtung (152) in Betriebsberührung mit dem ersten Ende des chromatographischen Mediums;
(iii) eine Absorptionseinrichtung (154) in Betriebsberührung mit dem zweiten Ende des chromatographischen Mediums; und
(iv) ein Detektoraufbringungskissen (156) in direkter Berührung mit der Leitungseinrichtung und einer derartigen Angeordnung, daß es in indirekter Berührung mit dem ersten Ende des chromatographischen Mediums steht; und
(b) ein zweiter gegenüberstellbarer Bestandteil (144), der eine Probenvorbereitungseinrichtung einschließt, die zur Aufnahme einer zu untersuchenden Probe ausgebildet ist, und die ein Probenvorbereitungskissen (158) beeinhaltet; wobei ein In-Gegenüberstellung-Bringen der ersten und zweiten gegenüberstellbaren Bestandteile bewirkt, daß das Probenaufbringungskissen die zu untersuchende Probe auf das Detektoraufbringungskissen und somit durch die Leitungseinrichtung auf das erste Ende des chromatographischen Mediums aufbringt.

6. Chromatographische Untersuchungsvorrichtung nach Anspruch 1,
wobei die Vorrichtung (180) folgendes umfaßt:
(a) einen ersten gegenüberstellbaren Bestandteil (182), der folgendes einschließt:
(i) ein chromatographisches Medium (186) mit ersten und zweiten Enden (188,190);
(ii) eine Absorptionseinrichtung (192) in Betriebsberührung mit dem zweiten Ende des chromatographischen Mediums; und
(iii) ein Detektoraufbringungskissen (194) in direkter Berührung mit dem ersten Ende des chromatographischen Mediums; und
(b) einen zweiten gegenüberstellbaren Bestandteil, der eine Probevorbereitungseinrichtung einschließt, die zur Aufnahme einer zu untersuchenden Probe ausgebildet ist und die ein Probenaufbringungskissen (196) beeinhaltet;
wobei die ersten und zweiten gegenüberstellbaren Bestandteile in Gegenüberstellung gebracht werden, wobei das Detektoraufbringungskissen und das Probenaufbringungskissen in Berührung stehen, außer in dem Bereich des Detektoraufbringungskissens, der direkt an das erste Ende des chromatographischen Mediums angrenzt, und wodurch ein In-Gegenüberstellung-Bringen der ersten und zweiten gegenüberstellbaren Bestandteile bewirkt, daß das Probenaufbringungskissen die zu testende Probe auf das Detektoraufbringungskissen und somit auf das erste Ende des chromatographischen Mediums aufbringt.

7. Chromatographische Untersuchungsvorrichtung nach Anspruch 1,
bei der die Vorrichtung (120) folgendes umfaßt:
(a) einen ersten gegenüberstellbaren Bestandteil (121), der folgendes einschließt:
(i) ein chromatographisches Medium (123) mit ersten und zweiten Enden (124,125);
(ii) eine Leitungseinrichtung (126), die derartig angeordnet ist, daß sie nicht in Betriebsberührung mit dem ersten Ende des chromatographischen Mediums stehen, wenn der erste gegenüberstellbare Bestandteil und der zweite gegenüberstellbare Bestandteil nicht in Gegenüberstellung sind; und
(iii) eine Absorptionseinrichtung (127) in Betriebsberührung mit dem zweiten Ende des chromatographischen Mediums; und
(b) ein zweiter gegenüberstellbarer Bestandteil (122) mit:
(i) einer ersten Aufbringungseinrichtung (130), einschließlich einer Probenvorbereitungseinrichtung, die zur Aufnahme einer zu untersuchenden Probe ausgebildet ist; und
(ii) eine zweite Aufbringungseinrichtung (131);
wobei die ersten und zweiten Aufbringungseinrichtungen auf dem zweiten gegenüberstellbaren Bestandteil derartig angeordnet sind, daß sie nicht in Betriebsberührung sind, wenn die ersten und zweiten gegenüberstellbaren Bestandteile nicht in Gegenüberstellung sind;
wobei ein In-Gegenüberstellung-Bringen der ersten und zweiten gegenüberstellbaren Bestandteile die Leitungseinrichtung in Betriebsberührung mit der ersten Aufbringungseinrichtung bringt, die Leitungseinrichtung mit der zweiten Aufbringungseinrichtung in Betriebsberührung bringt und die zweite Aufbringungseinrichtung mit dem ersten Ende des chromatographischen Mediums in Betriebsberührung bringt, wodurch die ersten und zweiten Aufbringungseinrichtungen miteinander in Betriebsberührung gebracht werden, um den Inhalt der ersten und zweiten Aufbringungseinrichtungen auf das chromatographische Medium aufzubringen.

8. Chromatographische Untersuchungsvorrichtung nach Anspruch 2, 3, 4, 5, 6 oder 7,
bei der das chromatographische Medium weiterhin eine Erfassungszone (20;106;128;162;202;418) einschließt, die eine wesentlich kleinere Fläche als das chromatographische Medium aufweist.

9. Chromatographische Untersuchungsvorrichtung nach Anspruch 8,
bei der die Erfassungszone einen spezifischen Bindungspartner für den am chromatographischen Medium immobilisierten Analyten einschließt.

10. Chromatographische Untersuchungsvorrichtung nach Anspruch 8,
bei der das chromatographische Medium weiterhin eine zentrale Zone (22;108;129;164;204;420) einschließt, die wesentlich kleiner als das chromatographische Medium ist und von der Erfassungszone getrennt ist.

11. Chromatographische Untersuchungsvorrichtung nach Anspruch 10,
bei der die Kontrollzone einen auf ihr immobilisierten Analyten einschließt.

12. Chromatographische Untersuchungsvorrichtung nach Anspruch 2,
bei der die Vorrichtung weiterhin zumindest eines der nachstehenden Teile umfaßt:
(i) eine Leitungseinrichtung (35) in Betriebsberührung mit dem ersten Ende des chromatographischen Mediums und
(ii) eine Absorptionseinrichtung (36) in Betriebsberührung mit dem zweiten Ende des chromatographischen Mediums.

13. Chromatographische Untersuchungsvorrichtung nach Anspruch 2,
bei der die Probenvorbereitungseinrichtung weiterhin einen für den Analyten spezifischen Bindungspartner enthält, der mit einer erfaßbaren Markierung in einer Form markiert ist, daß er durch Zusatz einer wässrigen Flüssigkeit zur Probenvorbereitungseinrichtung wieder aufgelöst werden kann.

14. Chromatographische Untersuchungsvorrichtung nach Anspruch 2,
bei der die Probenvorbereitungseinrichtung zumindest ein Reagenz zur Behandlung der Probe vor dem Aufbringen der Probe auf das chromatographische Medium enthält.

15. Chromatographische Untersuchungsvorrichtung nach Anspruch 2,
bei der die ersten und zweiten gegenüberstellbaren Bestandteile jeweils weiterhin Eingriffseinrichtungen (26,28) zur Befestigung der ersten und zweiten gegenüberstellbaren Bestandteile in Gegenüberstellung einschließen.

16. Chromatographische Untersuchungsvorrichtung nach Anspruch 7,
bei der die Probenvorbereitungseinrichtung ein Probenaufbringungskissen (428) einschließt, das zumindest ein Reagenz zur Behandlung der Probe vor dem Aufbringen der Probe auf das chromatographische Medium einschließt.

17. Chromatographische Untersuchungsvorrichtung nach Anspruch 3,
bei der die erste Aufbringungseinrichtung (428) einen ersten für den Analyten spezifischen Bindungspartner in einer Form enthält, die durch Zugabe einer wässrigen Probe zur Aufbringungseinrichtung wieder aufgelöst werden kann.

18. Chromatographische Untersuchungsvorrichtung nach Anspruch 3,
bei der die zweite Flüssigkeit einen zum Analyten spezifischen Bindungspartner umfaßt und das chromatographische Medium weiterhin eine Erfassungszone (418) umfaßt, die einen zweiten spezifischen Bindungspartner enthält, wobei der zweite spezifische Bindungspartner für den ersten spezifischen Bindungspartner spezifisch ist.

19. Chromatographische Untersuchungsvorrichtung nach Anspruch 4,
bei der die Probenvorbereitungseinrichtung ein Probenaufbringungskissen (110) einschließt und die zweite Aufbringungseinrichtung ein Detektoraufbringungskissen (112) einschließt, auf das das Erfassungsreagenz aufgebracht werden kann, wodurch, wenn die ersten und zweiten gegenüberstellbaren Bestandteile in Gegenüberstellung gebracht werden, der Inhalt des Probenaufbringungskissens und des Detektoraufbringungskissens auf die Leitungseinrichtung (102) aufgebracht wird, wobei das Detektoraufbringungskissen einen ersten zum Analyten spezifischen Bindungspartner in einer Form enthält, daß er durch Zugabe einer wässrigen Flüssigkeit auf das Detektoraufbringungskissen wieder aufgelöst werden kann, wobei der erste spezifische Bindungspartner mit einer erfaßbaren Markierung markiert ist, und das chromatographische Medium weiterhin eine Erfassungszone (106) umfaßt, deren Fläche wesentlich kleiner als das chromatographische Medium ist, wobei die Erfassungszone einen zweiten, darauf immobilisierten, zum Analyten spezifischen Bindungspartner enthält, derartig, daß ein Dreifachkomplex mit dem ersten spezifischen Bindungspartner, dem Analyten und dem zweiten spezifischen Bindungspartner auf der Erfassungszone gebildet wird, wenn der Analyt in der Probe vorhanden ist.

20. Chromatographische Untersuchungsvorrichtung nach Anspruch 5 oder 6,
bei der das Detektoraufbringungskissen (156;194) einen ersten zum Analyten spezifischen Bindungspartner in einer Form enthält, die durch Zusatz einer wässrigen Flüssigkeit zum Detektoraufbringungskissen wieder aufgelöst werden kann, wobei der erste spezifische Bindungspartner mit einer erfaßbaren Markierung markiert ist, und das chromatographische Medium weiterhin eine Erfassungszone (162;202) umfaßt, die eine im wesentlichen kleinere Fläche als das chromatographische Medium aufweist, wobei die Erfassungszone darauf immobilisiert, einen zweiten, zum Analyten spezifischen Bindungspartner enthält, derartig, daß ein Dreifachkomplex mit dem ersten spezifischen Bindungspartner, dem Analyten und dem zweiten spezifischen Bindungspartner auf der Erfassungszone gebildet wird, wenn der Analyt in der Probe vorhanden ist.

21. Chromatographische Untersuchungsvorrichtung nach Anspruch 4,
bei der die Probenvorbereitungseinrichtung ein Probenaufbringungskissen (130) einschließt und die zweite Aufbringungseinrichtung ein Detektoraufbringungskissen (131) einschließt, auf das Erfassungsreagenz aufgebracht werden kann, wodurch, wenn die ersten und zweiten gegenüberstellbaren Bestandteile in Gegenüberstellung gebracht werden, der Inhalt des Probenaufbringungskissens und des Detektoraufbringungskissens auf das chromatographische Medium aufgebracht wird, wobei das Detektoraufbringungskissen einen ersten zum Analyten spezifischen Bindungspartner in einer Form enthält, die durch Zugabe einer wässrigen Flüssigkeit zum Detektoraufbringungskissen wieder aufgelöst werden kann, wobei der erste spezifische Bindungspartner mit einer erfaßbaren Markierung markiert ist, und das chromatographische Medium weiterhin eine Erfassungszone (128) umfaßt, die eine wesentlich kleinere Fläche als das chromatographische Medium aufweist, wobei die Erfassungszone einen darauf immobilisierten zweiten, zum Analyten spezifischen Bindungspartner enthält, derartig, daß ein Dreifachkomplex mit dem ersten spezifischen Bindungspartner, dem Analyten und dem zweiten spezifischen Bindungspartner an der Erfassungszone gebildet wird, wenn der Analyt in der Probe vorhanden ist.

22. Chromatographische Untersuchungsvorrichtung nach Anspruch 13, 19, 20 oder 21,
bei der die erfaßbare Markierung eine visuell erfaßbare Markierung ist.

23. Chromatographische Untersuchungsvorrichtung nach Anspruch 4,
bei der der Analyt menschliches Hämoglobin ist, und der spezifische Bindungspartner ein Antikörper gegen menschliches Hämoglobin ist.

24. Ein Testsatz zur Erfassung und/oder Bestimmung eines Analyten mit:
(a) einer chromatographischen Untersuchungsvorrichtung nach Anspruch 2, 4, 5, 6 oder 7; und
(b) einen für den Analyten spezifischen Bindungspartner, der mit einer erfaßbaren Markierung markiert ist.

25. Ein Testsatz zur Erfassung und/oder Bestimmung eines Analyten mit:
(a) der chromatographischen Untersuchungsvorrichtung nach Anspruch 3; und
(b) einem spezifischen Bindungspartner für den ersten zum Analyten spezifischen Bindungspartner, der mit einer erfaßbaren Markierung markiert ist.

26. Testsatz nach Anspruch 24 oder 25,
bei dem die erfaßbare Markierung eine visuell erfaßbare Markierung ist.

27. Testsatz zur Erfassung und/oder Bestimmung eines Analyten mit:
(a) der chromatographischen Untersuchungsvorrichtung nach den Ansprüchen 4, 5, 6 oder 7; und
(b) einer wässrigen Flüssigkeit, um den für den Analyten spezifischen Bindungspartner, der mit einer erfaßbaren Markierung markiert ist, wieder auflösen zu können, die auf das Detektoraufbringungskissen aufgebracht werden soll.

28. Testsatz nach Anspruch 24, 25, 26 oder 27,
bei dem der zu erfassende Analyt menschliches Hämoglobin ist.

## Revendications

1. Dispositif d'analyse chromatographique pour la détection et/ou la détermination d'au moins un analyte comprenant au moins des premier et second composants opposables, l'un des premier et second composants opposables comprenant un milieu chromatographique et l'un des premier et second composants opposables comprenant un moyen de préparation de l'échantillon, le composant opposable comprenant le milieu chromatographique et le composant opposable comprenant le moyen de préparation de l'échantillon étant différents composants opposables, où les premier et second composants opposables peuvent être mis en opposition afin de forcer un liquide échantillon du moyen de préparation de l'échantillon à être appliqué au composant comprenant le milieu chromatographique afin de contacter ledit milieu chromatographique.

2. Dispositif d'analyse chromatographique de la revendication 1 où le dispositif (10) comprend :
(a) un premier composant opposable (14) comprenant un milieu chromatographique (18) ayant des première et seconde extrémités (19, 20) ; et
(b) un second composant opposable (12) comprenant un moyen de préparation de l'échantillon (16) adapté à recevoir un échantillon à analyser ; où les premier et second composants opposables peuvent être mis en opposition afin de forcer le moyen de préparation de l'échantillon à appliquer l'échantillon à tester au milieu chromatographique.

3. Dispositif d'analyse chromatographique de la revendication 1 où le dispositif (400) comprend :
(a) un premier composant opposable (402) comprenant :
(i) un milieu chromatographique (412) ayant des première et seconde extrémités (414, 416) ;
(ii) un premier moyen conducteur (422) en contact de fonctionnement avec la première extrémité du milieu chromatographique ; et
(iii) un second moyen conducteur (424) en contact de fonctionnement avec la seconde extrémité du milieu chromatographique ;
(b) un deuxième composant opposable (404) attaché de façon articulée au premier composant opposable et comprenant :
(i) un premier moyen d'absorption (426) ; et
(ii) un premier moyen d'application (428) séparé du premier moyen d'absorption et contenant un moyen de préparation de l'échantillon adapté à recevoir un échantillon à analyser ;
où la mise en opposition des premier et deuxième composants opposables force le premier moyen d'absorption à venir en contact de fonctionnement avec le second moyen de conducteur pour retirer le fluide du milieu chromatographique et force le premier moyen d'application à venir en contact de fonctionnement avec le premier moyen conducteur pour appliquer du fluide au milieu chromatographique de manière qu'un premier liquide appliqué au premier moyen d'application soit attiré à travers au moins une portion du milieu chromatographique ; et
(c) un troisième composant opposable (406) attaché de façon articulée au premier composant opposable et comprenant :
(i) un second moyen d'absorption (434) ; et
(ii) un second moyen d'application (432) séparé du second moyen d'absorption ;
où la mise en opposition des premier et troisième composants opposables forcent le second moyen d'absorption à venir en contact de fonctionnement avec le premier moyen conducteur pour retirer du fluide du milieu chromatographique et force le second moyen d'application à venir en contact de fonctionnement avec le second moyen conducteur pour appliquer du fluide au milieu chromatographique de manière qu'un second liquide appliqué au second moyen d'application soit attiré à travers au moins une portion du milieu chromatographique recouvrant la portion du milieu chromatographique par où est attiré le premier liquide ; les premier, deuxième et troisième composants opposables étant en une configuration telle que quand le troisième composant opposable est mis en opposition avec le premier composant opposable, le deuxième composant opposable puisse être replié sur les premier et troisième composants opposables pour former un couvercle.

4. Dispositif d'analyse chromatographique de la revendication 1 où le dispositif (90) comprend :
(a) un premier composant opposable (92) comprenant :
(i) un milieu chromatographique (96) ayant des première et seconde extrémités (98, 100) ;
(ii) un moyen conducteur (102) en contact de fonctionnement avec la première extrémité du milieu chromatographique ; et
(iii) un moyen d'absorption (104) en contact de fonctionnement avec la seconde extrémité du milieu chromatographique ; et
(b) un second composant opposable (94) comprenant :
(i) un premier moyen d'application (110) comprenant un moyen de préparation de l'échantillon adapté à recevoir un échantillon à analyser ; et
(ii) un second moyen d'application (112), les premier et second moyens d'application étant placés sur le second composant opposable de manière à ne pas être en contact de fonctionnement quand les premier et second composants opposables ne sont pas en opposition ; où la mise des premier et second composants opposables en opposition place le moyen conducteur en contact de fonctionnement avec le premier moyen d'application et place le moyen conducteur en contact de fonctionnement avec le second moyen d'application, plaçant ainsi les premier et second moyens d'application en contact de fonctionnement l'un avec l'autre.

5. Dispositif d'analyse chromatographique de la revendication 1 où le dispositif (140) comprend :
(a) un premier composant opposable (142) comprenant :
(i) un milieu chromatographique (146) ayant des première et seconde extrémités (148, 150) ;
(ii) un moyen conducteur (152) en contact de fonctionnement avec la première extrémité du milieu chromatographique ;
(iii) un moyen d'absorption (154) en contact de fonctionnement avec la seconde extrémité du milieu chromatographique ; et
(iv) un tampon d'application d'un détecteur (156) en contact direct avec le moyen conducteur et placé de manière qu'il soit en contact direct avec la première extrémité du milieu chromatographique ; et
(b) un second composant opposable (144) comprenant un moyen de préparation de l'échantillon adapté à recevoir un échantillon à analyser comprenant un tampon de préparation de l'échantillon (158) ; et la mise des premier et second composants opposables en opposition force le tampon d'application de l'échantillon à appliquer l'échantillon à tester au tampon d'application du détecteur et ainsi à la première extrémité du milieu chromatographique à travers le moyen conducteur.

6. Dispositif d'analyse chromatographique de la revendication 1 où le dispositif (180) comprend :
(a) un premier composant opposable (182) comprenant :
(i) un milieu chromatographique (186) ayant des première et seconde extrémités (188, 190) ;
(ii) un moyen d'absorption (192) en contact de fonctionnement avec la seconde extrémité du milieu chromatographique ; et
(iii) un tampon d'application d'un détecteur (194) en contact direct avec la première extrémité du milieu chromatographique ; et
(b) un second composant opposable comprenant un moyen de préparation de l'échantillon adapté à recevoir un échantillon à analyser comprenant un tampon d'application de l'échantillon (196) ;
où les premier et second composants opposables sont mis en opposition, le tampon d'application du détecteur et le tampon d'application de l'échantillon sont en contact à l'exception de la région du tampon d'application du détecteur qui est directement adjacente à la première extrémité du milieu chromatographique et la mise des premier et second composants opposables en opposition force le tampon d'application de l'échantillon à appliquer l'échantillon à tester au tampon d'application du détecteur et ainsi à la première extrémité du support chromatographique.

7. Dispositif d'analyse chromatographique de la revendication 1 où le dispositif (120) comprend :
(a) un premier composant opposable (121) comprenant :
(i) un milieu chromatographique (123) ayant des première et seconde extrémités (124, 125) ;
(ii) un moyen conducteur (126) placé de manière qu'il ne soit pas en contact de fonctionnement avec la première extrémité du milieu chromatographique quand le premier composant opposable et le second composant opposable ne sont pas en opposition ; et
(iii) un moyen d'absorption (127) en contact de fonctionnement avec la seconde extrémité du milieu chromatographique ; et
(b) un second composant opposable (122) comprenant :
(i) un premier moyen d'application (130) comprenant un moyen de préparation de l'échantillon adapté à recevoir un échantillon à analyser ;
(ii) un second moyen d'application (131) ;
les premier et second moyens d'application étant placés sur le second composant opposable de façon qu'ils ne soient pas en contact de fonctionnement quand les premier et second composants opposables ne sont pas en opposition, où la mise des premier et second composants opposables en opposition place le moyen conducteur en contact de fonctionnement avec le premier moyen d'application, place le moyen conducteur en contact de fonctionnement avec le second moyen d'application et place le second moyen d'application en contact de fonctionnement avec la première extrémité du milieu chromatoqraphique, plaçant ainsi les premier et second moyens d'application en contact de fonctionnement l'un avec l'autre pour appliquer les contenus des premier et second moyens d'application au support chromatographique.

8. Dispositif d'analyse chromatographique de la revendication 2, 3, 4, 5, 6 ou 7, où le milieu chromatographique comprend de plus une zone de détection (20 ; 106 ; 128 ; 162 ; 202 ; 418) sensiblement plus petite en aire que le milieu chromatographique.

9. Dispositif d'analyse chromatographique de la revendication 8 où la zone de détection comprend un partenaire de liaison spécifique pour l'analyte immobilisé sur le milieu chromatographique.

10. Dispositif d'analyse chromatographique de la revendication 8 où le milieu chromatographique comprend de plus une zone centrale (22 ; 108 ; 129 ; 164 ; 204 ; 420) sensiblement plus petite que le milieu chromatographique et séparée de la zone de détection.

11. Dispositif d'analyse chromatographique de la revendication 10 où la zone de contrôle comprend un analyte qui y est immobilisé.

12. Dispositif d'analyse chromotographique de la revendication 2 où le dispositif comprend de plus au moins l'un de :
(i) un moyen conducteur (35) en contact de fonctionnement avec la première extrémité du milieu chromatographique et
(ii) un moyen d'absorption (36) en contact de fonctionnement avec la seconde extrémité du milieu chromatographique.

13. Dispositif d'analyse chromatographique de la revendication 2 où le moyen de préparation de l'échantillon contient de plus un partenaire de liaison spécifique pour l'analyte, marqué d'un marqueur détectable sous une forme qui peut être resolubilisée par l'addition d'un liquide aqueux au moyen de préparation de l'échantillon.

14. Dispositif d'analyse chromatographique de la revendication 2 où le moyen de préparation de l'échantillon contient au moins un réactif pour un traitement de l'échantillon avant que l'échantillon ne soit appliqué au milieu chromatographique.

15. Dispositif d'analyse chromatographique de la revendication 2 où les premier et second composants opposables comprennent chacun, de plus, un moyen d'engagement (26, 28) pour fixer les premier et second composants opposables en opposition.

16. Dispositif d'analyse chromatographique de la revendication 7 où le moyen de préparation de l'échantillon comporte un tampon d'application de l'échantillon (428) contenant au moins un réactif pour un traitement de l'échantillon avant que l'échantillon ne soit appliqué au support chromatographique.

17. Dispositif d'essai chromatographique de la revendication 3 où le premier moyen d'application (428) contient un premier partenaire de liaison spécifique pour l'analyte sous une forme qui peut être resolubilisée par l'addition d'un échantillon aqueux au moyen d'application.

18. Dispositif d'analyse chromatographique de la revendication 3 où le second liquide comprend un partenaire de liaison spécifique à l'analyte et le milieu chromatographique comprend de plus une zone de détection (418) contenant un partenaire de liaison spécifique secondaire, le partenaire de liaison spécifique secondaire étant spécifique du premier partenaire de liaison spécifique.

19. Dispositif d'analyse chromatographique de la revendication 4 où le moyen de préparation de l'échantillon comprend un tampon (110) d'application de l'échantillon et le second moyen d'application comprend un tampon d'application d'un détecteur (112) auquel peut être appliqué un réactif détecteur, ainsi, quand les premier et second composants opposables sont mis en opposition, les contenus du tampon d'application de l'échantillon et du tampon d'application du détecteur sont appliqués au moyen conducteur (102), le tampon d'application du détecteur contenant un premier partenaire de liaison spécifique à l'analyte sous une forme qui peut être resolubilisée par addition d'un liquide aqueux au tampon d'application du détecteur, le premier partenaire de liaison spécifique étant marqué d'un marqueur détectable et le milieu chromatographique comprend de plus une zone de détection (106) sensiblement plus petite en aire que le milieu chromatographique, la zone de détection contenant un second partenaire de liaison spécifique à l'analyte qui y est immobilisé de manière qu'un complexe ternaire comprenant le premier partenaire de liaison spécifique, l'analyte, et le second partenaire de liaison spécifique se forme à la zone de détection si l'analyte est présent dans l'échantillon.

20. Dispositif d'essai chromatographique de la revendication 5 ou 6 où le tampon d'application du détecteur (156, 194) contient un premier partenaire de liaison spécifique à l'analyte sous une forme qui peut être resolubilisée par addition d'un liquide aqueux au tampon d'application du détecteur, le premier partenaire de liaison spécifique étant marqué par un marqueur détectable et le milieu chromatographique comprend de plus une zone de détection (162 ; 202) sensiblement plus petite en aire que le milieu chromatographique, la zone de détection contenant un second partenaire de liaison spécifique à l'analyte immobilisé de manière qu'un complexe ternaire comprenant le premier partenaire de liaison spécifique, l'analyte et le second partenaire de liaison spécifique se forme à la zone de détection si l'analyte est présent dans l'échantillon.

21. Dispositif d'analyse chromatographique de la revendication 4 où le moyen de préparation de l'échantillon comprend un tampon d'application de l'échantillon (130) et le second moyen d'application comprend un tampon d'application du détecteur (131), où peut être appliqué le réactif de détection, ainsi quand les premier et second composants opposables sont mis en opposition, les contenus du tampon d'application de l'échantillon et du tampon d'application du détecteur sont appliqués au milieu chromatographique, le tampon d'application du détecteur contenant un premier partenaire de liaison spécifique à l'analyte sous une forme qui peut être resolubilisée par addition d'un liquide aqueux au tampon d'application du détecteur, le premier partenaire de liaison spécifique étant marqué d'un marqueur détectable et le milieu chromatographique comprend de plus une zone de détection (128), sensiblement plus petite en aire que le milieu chromatographique, la zone de détection contenant un second partenaire de liaison spécifique à l'analyte immobilisé, de manière qu'un complexe ternaire comprenant le premier partenaire de liaison spécifique, l'analyte, et le second partenaire de liaison spécifique se forme à la zone de détection si l'analyte est présent dans l'échantillon.

22. Dispositif d'analyse chromatographique de la revendication 13, 19, 20 ou 21 où le marqueur détectable est un marqueur visuellement détectable.

23. Dispositif d'analyse chromatographique de la revendication 4 où l'analyte est l'hémoglobine humaine et le partenaire de liaison spécifique est un anticorps anti-hémoglobine humaine.

24. Trousse de test pour la détection et/ou la détermination d'un analyte comprenant :
(a) un dispositif d'analyse chromatographique de la revendication 2, 4, 5, 6 ou 7 ; et
(b) un partenaire de liaison spécifique pour l'analyte marqué d'un marqueur détectable.

25. Trousse de test pour la détection et/ou la détermination d'un analyte comprenant :
(a) le dispositif d'analyse chromatographique de la revendication 3 ; et
(b) un partenaire de liaison spécifique pour le premier partenaire de liaison spécifique à l'analyte marqué par un marqueur détectable.

26. Trousse de test de la revendication 24 ou 25 où le marqueur détectable est un marqueur visuellement détectable.

27. Trousse de test pour la détection et/ou la détermination d'un analyte comprenant :
(a) le dispositif d'analyse chromatographique des revendications 4, 5, 6 ou 7 ; et
(b) un liquide aqueux pour la resolubilisation du partenaire de liaison spécifique pour l'analyte marqué par un marqueur détectable, à appliquer au tampon d'application du détecteur.

28. Trousse de test de la revendication 24, 25, 26 ou 27, où l'analyte à détecter est l'hémoglobine humaine.
